(19) Europäisches Patentamt / European Patent Office / Office européen des brevets

(11) EP 3 875 377 B1

(12) EUROPEAN PATENT SPECIFICATION

(45) Date of publication and mention
of the grant of the patent:
**09.07.2025 Bulletin 2025/28**

(21) Application number: **19879123.8**

(22) Date of filing: **30.10.2019**

(51) International Patent Classification (IPC):
**B65B 1/08** *(2006.01)* **B65B 1/32** *(2006.01)*
**B65B 31/02** *(2006.01)*

(52) Cooperative Patent Classification (CPC):
**B65B 61/00;** B65B 1/32; B65B 7/2821

(86) International application number:
**PCT/ES2019/070740**

(87) International publication number:
**WO 2020/089503 (07.05.2020 Gazette 2020/19)**

(54) **PROCEDURE FOR THE FILLING OF SOLIDS IN PHARMACEUTICAL CONTAINERS AND THE SEALING THEREOF UNDER STERILE CONDITIONS**

VERFAHREN ZUM ABFÜLLEN VON FESTSTOFFEN IN PHARMAZEUTISCHE BEHÄLTER UND DEREN VERSIEGELUNG UNTER STERILEN BEDINGUNGEN

PROCÉDÉ POUR LE REMPLISSAGE DE SOLIDES DANS DES CONTENANTS PHARMACEUTIQUES ET LE SCELLEMENT DE CEUX-CI DANS DES CONDITIONS STÉRILES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA TN**

(30) Priority: **02.11.2018 ES 201831060**

(43) Date of publication of application:
**08.09.2021 Bulletin 2021/36**

(73) Proprietor: **Laboratorios Farmacéuticos Rovi, S.A.**
**28037 Madrid (ES)**

(72) Inventors:
• **GUTIERRO ADURIZ, Ibón**
**28037 Madrid (ES)**
• **GARCÍA AMO, María**
**28037 Madrid (ES)**
• **CEBADERA MIRANDA, Elena**
**28037 Madrid (ES)**

(74) Representative: **Elzaburu S.L.P.**
**Edificio Torre de Cristal**
**Paseo de la Castellana 259 C, planta 28**
**28046 Madrid (ES)**

(56) References cited:
EP-A1- 2 078 674    WO-A1-2014/189965
WO-A1-2018/017561    WO-A2-2016/185230
CN-U- 207 768 787    GB-A- 2 271 347
US-A- 2 524 560    US-A- 6 065 270
US-A1- 2017 028 130

## Description

## Field of the Invention

The present invention falls within the field of filling and sealing under sterile conditions of pharmaceutical containers including syringes, vials, capsules, ampoules, single-dose devices or cartridges that have been filled with solid substances selected from the group formed by powder, granules, pellets, nanoparticles, or microparticles, obtaining the sealing of these solid substances. More particularly, the field of the invention relates to a procedure for the filling and sealing of pharmaceutical containers which have been filled with one or more sterile solid pharmaceutical substances or sterile excipients dispensed and prepared in an aseptic environment which avoids adherence of the said substances to the sides of the pharmaceutical containers, thus ensuring the tightness of the container seal.

## Background Art

In the pharmaceutical industry, the filling process of pharmaceutical containers is often carried out with liquid pharmaceutical substances and/or freeze-dried solids, as these are much easier to handle and involve fewer dosing problems than solids such as powders, granules, pellets, nanoparticles, microparticles, and others. The use of solids such as those mentioned above in the container filling process has the major drawback that these solids tend to adhere to the walls or body of the containers, preventing or at least hindering the obtaining of the necessary tightness in the container seal. This adherence to the walls or to the body, in addition to preventing the desired tightness, leads to contamination of the containers and loss of doses, as containers in which such adherence to the walls is observed must be discarded because as part of the solid remains in the sealing area of the container walls, it is not possible to know the exact amount of solid that will be delivered to the patient. On the other hand, with regard to contamination, as the dispensed solid remains adhered to the walls of the sealing area, the stopper used to seal the container does not close hermetically, so it will not be able to prevent the entry of substances from the environment into the container and will not ensure the integrity of the product, such that its physico-chemical and microbiological properties may vary, affecting the quality of the medicinal product. This is the biggest drawback that the pharmaceutical industry can encounter in this field due to the strict conditions imposed by the regulations of the industry, which must also comply with the standards known as Good Manufacturing Practices (GMPs).

Another concern for the pharmaceutical industry is ensuring the integrity of the closure, which also affects safety, as small losses of the medicine can affect the safety of the healthcare workers handling it. The term integrity here refers to the physic ability of a container closure system to maintain product sterility and quality of final sterile pharmaceutical, biological and vaccine products throughout their lifetime. A sterile product is also defined as a product free of micro-organisms, whose composition is one or more of the elements exposed to aseptic conditions and which ultimately make up the sterile finished pharmaceutical product. These elements include the containers, closures, and components of the finished pharmaceutical product.

When dosing powder into pharmaceutical containers, several factors affecting the cleanliness of the inner walls of the containers must be considered, as lack of cleanliness results in contamination. These factors are listed below:

- The static charge of the walls of the pharmaceutical containers used for filling, as well as the static charge of the solid that is dispensed into them: If the wall and solid loads have opposite charges, the dispensed solid will adhere to the container walls.

- The kinetic energy that both the dispensed solid and the elements in contact with it acquire when the solid falls into the containers: The greater the height from which the solid to be dispensed falls freely to the bottom of the containers, the greater the kinetic energy acquired by the solid and the elements due to friction.

The length of the dispenser needles (also known as nozzles) used for dosing, as the longer the dispenser is and the closer it is to the top of the dispensed solid in the container, the less kinetic energy it will have. In addition, the dispenser conveys the dispensed solid to an area away from the wall surfaces of the container used. The ideal distance between the dispensed solid and the tip of the dispenser will depend on the dispensing rate and the density of the solid dispensed.

- Redirection of the air displaced inside the containers. This phenomenon is related to the kinetic energy of the dispensed solid when it is released into the container. During dispensing, the inrush of the solid into the container displaces the air inside the container upwards. This displaced air is full of suspended particles. Thus, the dispenser can be thought of as a "chimney" that moves the air stream away from the interior walls, preserving them from this contamination.

- The use of large airflow streams (unidirectional or turbulent regime) in filling cabins or filling sites as required by international Pharmacopoeias to ensure the removal of any foreign particles that may contaminate the final product from the aseptic filling and sealing process. The use of these airflow streams makes filling with solids quite difficult, as a disturbance is generated that causes the solid to adhere to the walls of the container used for filling.

In order to eliminate the adherence of the solid to the walls of the container, one of the measures used is to perform a process of ionisation of both the container and the solid to be filled in it. In the present invention, the terms "process", "stage" and "phase" are used interchangeably, as well as the terms "ionisation" and "deionisation" or "ioniser" and "deioniser".

Ionisation is a chemical or physical phenomenon by which ions are produced. Ions are atoms or molecules that are electrically charged due to an excess or lack of electrons relative to a neutral atom or molecule. The chemical species with more electrons than the neutral atom or molecule is called an anion, having a net negative charge, and the one with fewer electrons is called a cation, having a net positive charge.

The ionisation process employed in the present invention is used both to neutralise the electrostatic charge of the pharmaceutical container to be filled with the pharmaceutical solid, and to neutralise the electrostatic charge of the solid to be dispensed, i.e., for both the container and the contents. This ionisation is also used to neutralise the elements of the dosing and capping equipment that come into contact with the container and/or the powder. For this purpose, the ioniser generates ions of both polarities which are projected onto the surface of the object to be neutralised, where ions of opposite signs are recombined and those of the same sign are rejected. Throughout this document, "ioniser" means any element or device which is capable of ionising the surrounding air molecules, so that they are then projected onto a surface which has static electrical charges that neutralise those charges, thereby ionising that surface.

However, with this ionisation process only it is not possible to avoid the serious problem of adherence to the sides of the container during the process of filling the containers with solids, because when the solid is filled through the nozzle, the kinetic energy carried by the solid generates turbulence inside the container that eventually again causes part of the solid to stick to the walls or the body of the container.

As regards the prior art, the documents cited below describe the ionisation technique that results in the neutralisation of electrical charges applied to various situations:

In this regard, patent US 2016/0200461 A1 filed by VANRX Pharmasystems INC. describes a method for volumetric filling and aseptic sealing of containers such as vials, bottles, syringes and ampoules with a liquid pharmaceutical product (which can be subsequently lyophilised) in a controlled environment. This publication mentions concerns about the materials from which the containers are made, whether glass or polymeric materials. On one hand, glass containers suffer from breakage, scratching, and particle emission due to collisions between them. On the other hand, containers made of polymeric materials are more resistant than those made of glass, although they suffer from cosmetic defects such as scratches, which can impair the quality of the pharmaceutical product due to collisions.

A substantial difference in the present invention with respect to the document cited is that the compounds handled are solid substances that are much more difficult to dose, since they are highly charged and have a higher specific surface area. Moreover, in the above-mentioned document the sealing process comprises two stages, a partial stage and a second complete stage due to the need for freeze-drying after the first partial sealing, whereas in the present invention the sealing process is carried out in a single stage with a complete sealing, without the need to resort to subsequent sealing stages.

In addition, it should be noted that the filling process of solid substances is much more complex due to the fact that the solid substances remain adhered to the walls of the pharmaceutical containers, impairing the precision of the dosage, which is more relevant in small calibre containers where it is necessary to dose small quantities of medicines in a very precise way. This drawback is solved by the procedure proposed in the present invention, since it must be taken into account that, in the pharmaceutical industry, an error in the filling of the active ingredient may mean that patients will receive an incorrect dose of the product.

This is a serious drawback when filling containers with solids due to the problem of the adherence of the solid to the body of the containers. For this reason, in most of the procedures used today in the pharmaceutical industry, certification of the equipment is mandatory to ensure that the dispensed quantity is adequate. In addition, various in-process controls are incorporated during packaging to verify the actual filling quantity of all pharmaceutical containers. A common control is weighing the containers, which allows to rectify or discard containers in which the quantity of pharmaceutical substance, either drug or active ingredient, does not meet the required weighing accuracy. In-process controls can be 100% or statistical; the latter are carried out from time to time to check the dosage. These controls involve a high production and economic cost, which is necessary to control the precision of the product dosage.

As for the elimination of electrostatic charge, several types of ionisers are available for dealing with this problem. These ionisers come in various shapes, such as rod, gun, curtain, blade, barrel, needle, or filter ionisers, including isolators with an ioniser on the top of the isolator, among others. For the purpose of this invention, these ionisers can be installed on the machinery used for the filling process to produce ions of both polarities that neutralise the surface of the containers or products, or they can also be placed in the packaging areas, room or isolators, specifically on the ceiling of the isolators to produce an ionisation that neutralises both the environment and the air flow of the area, thereby eliminating the problem of static charges.

With regard to the removal of the static charge of solids by ionisers, several documents are cited below: European patent EP 2711096 A2 applied for by TRINC Corporation relates to a device for the removal of electrostatic charge and dirt from objects such as film, foil, glass, clothing, paper or the like. The device comprises a large container with an opening at the top and an opening at the bottom for suctioning and discharging the powder and a small cylindrical or conical container inside the large container. This small container is designed to generate a cyclone current and tornado current within it, and comprises at least one corona discharge ion generator. This ion generator consists of electric discharge needles that are placed either on top of or inside the small container. The small container comprises air injection openings through which compressed air is injected, as well as ultrasound generators inside or outside the small container meant to make the powder vibrate so that it can be separated from the desired object once it has been neutralised by the ion generator. This powder can be collected by vacuum suction inside the large container.

The present invention, instead, relates to the deionisation of both the container and the powder prior to packaging in order to prevent the powder from sticking to the container walls during the filling process and thus to achieve a complete seal. In addition, as a safety factor, deionisation is carried out inside the container to remove any remaining powder adhered to the walls of the sealing area.

The present invention also uses an ioniser, whether in the form of a rod, gun, curtain, blades, barrel, needle or nozzle, or an ionising filter, including isolators with an ioniser on the top of said isolator, to eliminate static electricity, but it does so both on the powdered solid and the container to be used, which is not done in European patent EP 2711096 A2, as this only ionises the powder and does not ionise the container. Another substantial difference of the present invention with said document is based on the fact that said European patent EP 2711096 uses compressed air to facilitate suction, whereas the present invention does not need an air stream and, if it does, it should be of a sterile carrier gas. Among sterile carrier gases, an ionised nitrogen stream has advantages which will be discussed below.

A characteristic drawback of the present invention is the need to carry out the deionisation in sterile environments, making the use of sterile carrier gases necessary. It should be noted that this sterile condition of the carrier gas does not affect the deionisation process.

On the other hand, European patent EP 2711096 A2 differs from the present invention in that, although in both it is important to eliminate the electrostatic charge of the solid, said document does not discuss in detail the method by which this is carried out, mentioning only the use of an ion generator such as discharge needles for deionisation, without mentioning the problem caused by the electric discharge needles when they approach any solid, namely the appearance of a combustion phenomenon burning the product, generating impurities and altering the physicochemical composition of the product.

Japanese patent JP 2005001818 A applied for by YMS KK refers to a powder supply device and an air conveying device capable of feeding easily-charged powder and charged powder. This device comprises a hopper equipped with aeration means that are in turn equipped with a microporous diaphragm to aerate the powder in the hopper. The air for aeration is pre-ionised by an ionisation device such as a corona discharge device. Compressed air supplied by an air compressor is used for aeration. This compressed air is ionised by an air ionisation device comprising a corona discharge device or the like. When the aeration is performed by ionised air, the ionised air neutralises or removes the surface charge of the powder, so that the surface charge of the charged powder disappears. In addition, when aeration causes the powder in the hopper to inflate air and a layer of air forms between the powder and the inner wall of the hopper, the powder is prevented from becoming charged again. In addition, this document refers to a suction nozzle or needle made of conductive material that forms part of the air conveying device. This nozzle is never a dosing nozzle.

In the present invention, on the other hand, the solid substance is ionised on one hand and the pharmaceutical container is ionised on the other hand, using a sterile carrier gas stream as the ionised gas, usually a stream of sterile nitrogen. Another difference of the present invention with this Japanese patent is in the nozzle or needle; in the case of this Japanese patent, it refers to a suction nozzle made of conductive material, whereas, in the present invention, the nozzle is a dispensing needle and is not made of conductive material. In contrast to this Japanese patent, the present invention also uses an ioniser, whether in the form of a rod, gun, curtain, blades, barrel, needle or nozzle, or an ionising filter, including isolators with an ioniser on the top of the isolator or of any other type, in order to neutralise the electrostatic charge of both the container to be filled and the solid to be dispensed, whereas the Japanese patent only mentions the use of a corona discharge ionising device or the like.

Chinese utility model CN 203265193U by Meech Static Eliminators Shanghai Co LTD makes reference to the technical field of static electricity and powder removal from the inner wall of bottles, prior to filling, for container cleaning, using for this purpose an ion needle with compressed air to eliminate static electricity and remove the powder adhering to the walls of the bottle. The device described in this utility model consists of a needle, a first tube connecting the needle and a second tube for connecting the electrical cable, the two ends of which have an internal thread or an external thread respectively. The needle tip and the second tube are screwed and fixed together, and the needle tip, the first tube and the second tube have an interconnected inner passage for the passage of air, and the second tube also has at least two threads, so that the passage thread is also connected to the inner bore of the first tube. The tube features uniformly spaced wire holes between the inner and outer wall, each of these holes forming an ion-generating end at one extreme of the second tube that fits into the first member of the tube. The ions can be introduced into the surface of the proposed object through the ion needle and with the help of compressed air.

In the present invention, instead, the ioniser can be of any type, as a rod, gun, curtain, blades, barrel, needle or nozzle, or even an ionising filter, among which can also be found isolators with an ioniser on the top of said isolator, without the need for it to be a needle as specifically mentioned in the Chinese utility model. Furthermore, this utility model is assisted by compressed air to displace the ions, whereas the present invention may or may not use a sterile carrier gas stream, which may consist of sterile compressed nitrogen or air, not only to facilitate the ionisation process by aiding the dosing of the solid but also to maintain the necessary sterile conditions required for these procedures in the pharmaceutical industry by generating an inert atmosphere inside the containers. On the other hand, the utility model relates to cleaning bottles with powder prior to filling, whereas the present invention relates to deionisation and cleaning of walls after filling and applies to smaller containers than bottles, such as syringes, vials, capsules, ampoules, single-dose devices or cartridges, which are more difficult to fill with a solid such as powder.

International patent WO 2016/185230 A2 filed by 3P Innovation Limited describes an apparatus and method for filling pharmaceutical containers such as syringes, vials, capsules, cartridges and blister packs with powdered pharmaceutical material by vibration. This apparatus has a support for the pharmaceutical container, a tank containing pharmaceutical powder, this tank being in contact with a nozzle or filling needle in charge of filling the pharmaceutical container with the pharmaceutical powder, and a piezo-electric vibration device. This document relates to the advantage of using a cylinder comprising an electrically conductive material, which can be grounded through the weighing cell, thereby helping dissipate the static charge of plastic pharmaceutical containers to achieve a better powder filling process without compromising cleanliness. However, the invention described in that document would not require the material to be electrically conductive, since it is a process for filling through the mouth of the container, so that the problem of sealing that occurs when filling from the back of the container would not arise.

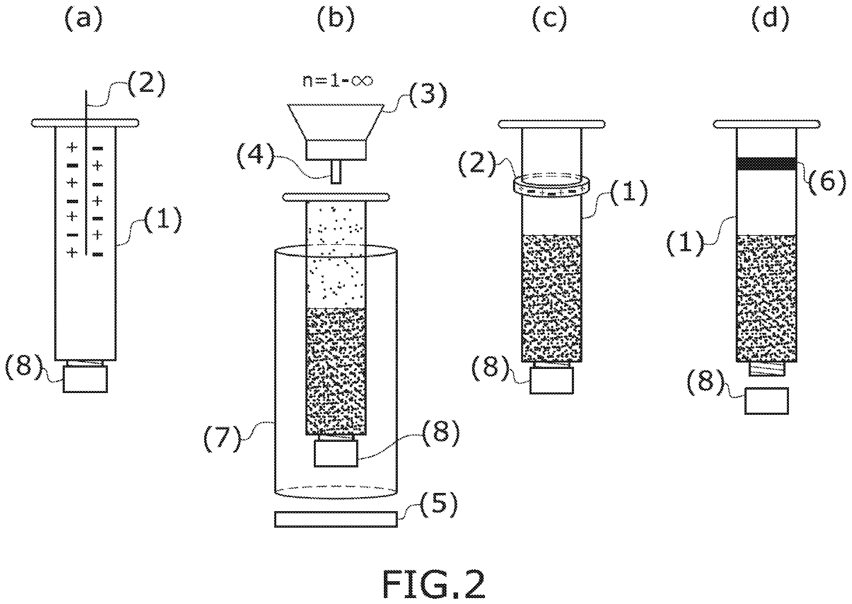

The present invention, on the other hand, concerns a process for sealing pharmaceutical containers which are filled with pharmaceutical solids, wherein such filling is carried out under aseptic conditions without the need for terminal sterilisation, whereas said international patent WO 2016/185230 A2 describes filling through the mouth of the container as can be seen in figure 2. On the other hand, this international publication only mentions the filling of plastic pharmaceutical containers such as containers, whereas the present invention includes all types of materials, such as polymeric materials or glass, for the container. Furthermore, the international publication mentions a cylinder or puck with an electrically conductive material meant to dissipate the static charge of the pharmaceutical containers to be used, whereas, in the present invention, the existence of a cylinder acting as a support for the container is an optional element, unrelated to the problem to be solved, and which is also intended for various other functions, such as:

- The use of such an element for the handling of the container without contact with same.
- The cylinder protects the process from air streams by being part of the "exclusion hood".
- It is an element for vertical support of the container on the weighing cell for accurate weighing.

Finally, WO2018/017561 in the name of Cutispharma discloses systems and methods for dispensing a powdered pharmaceutical into a bottle. Certain embodiments include static reduction devices such as one or more static bars.

Furthermore, in the present invention it is possible to use any ioniser, whatever form it may take, i.e. bar, gun, curtain, blades, barrels, needle or nozzle, or an ionising filter, among which can also be isolators with an ioniser on the top of said isolator which can be installed on the packaging equipment prior to filling, during filling and after filling, using or not a sterile carrier gas, whereas the invention described in said international publication only deals with a cylinder (puck) with electrically conductive material that can dissipate the static charge of the plastic container used during filling.

## Summary of the Invention

Accordingly, the problem to be solved in the present invention is to provide a procedure for filling pharmaceutical containers which may take the form of vials, capsules, ampoules, single-dose devices, inhalers, bottles, blister cartridges, sachets, bags, test tubes, Eppendorf® tubes and syringes. The syringes of the present invention may have a needle, catheter type cone, or Luer lock type cone, i.e. with an unthreaded tip or with female or male threaded tip, respectively. For the purpose of the present invention, "Luer cone" refers to the cone-shaped tip invented by Wülfing Luer with a typical taper of 6%, which can be male or female depending on the coupling. Likewise, "Luer lock cone" refers to the cone-shaped tip invented by the German Wülfing Luer with an airtight threaded closure.

The compounds of the containers of the present invention are materials such as plastics of different composition, such as polyolefins and cyclopolyolefins, polypropylene, polybutadiene, polyethylene, polystyrene, polyvinyl chloride, polyacrylonitrile, polyamides, etc., polyesters (containing the ester functional group in their main chain: poly(ethylene terephthalate), polycarbonate), acrylic polymers (poly(methyl methacrylate), polyacrylonitrile), thermoplastic resins (polyacetals and polyhaloethylenes), polyurethanes, formaldehyde resins (phenol resin, urea resin), phenoplasts, aminoplasts, thioplasts, duroplastic resins (unsaturated polyesters, polyurethanes), polyvinylidene silicones, cellulose derivatives, polycarbonates, and mixtures thereof, etc. Alternatively, the container can also be made of metal, e.g. steel or titanium suitable for drug delivery, glass, etc., with solids in sterile conditions that overcome the problems existing in the state of the art, and in particular that prevent the solid from adhering to the walls of the container, while ensuring an airtight sealing of the container.

In turn, both the cylinder or puck, the hopper and the nozzle or needle will preferably be composed of various non-conductive materials such as various plastics, e.g. polyether etherketone (PEEK), glass, stone, resin, glass, but may also be composed of grounded conductive materials such as steel or titanium, etc.

Both the materials used for the container and the cylinder materials must be watertight, inert, not very permeable or impermeable, that do not absorb and/or adsorb the contained product, not rough, and free of particles.

The solution to the problem described in the present invention is based on the fact that the inventors have found that such a problem can be satisfactorily solved by means of the following techniques, which can be applied independently or in any combination:

On the one hand, ionising both the solid and the pharmaceutical container where it is to be deposited, as well as ionising the elements of the dosing and capping equipment that comes into contact with the container and/or the powder, at one or more stages of the filling procedure, in order to prevent the solid from tending to adhere to the walls of the container, as well as the walls of the container from tending to attract the solid particles, so that the only tendency of the solid is to fall to the bottom of the container and not be deposited on its walls. This deionisation technique can be applied to the container and the solid separately, as well as to the container with product inside. This deionisation can be applied as many times as there are filling and capping steps in the process;

On the other hand, by controlling the potential applied to the ionisers, which should be such that the resulting electrostatic charge on the container walls and/or the dispensed solid is preferably less than 2,000 V, more preferably less than 500 V, and most preferably less than 200 V.

In addition, the filling of the pharmaceutical container with the solid is preferably carried out using a dispensing needle whose tip or dispensing end is located, throughout the filling stage, at a height between 1 to 3 mm above the surface of the solid deposited on the bottom of the container, so as to avoid turbulence that could lift the deposited solid towards the walls. Even if this turbulence phenomenon were to occur to some extent, ionisation of both the solid and the inner walls of the container will make said lifted particles settle back on the bottom of the container, without substantial loss of product on the container walls.

Consequently, in a first aspect the invention relates to a process for filling under sterile conditions pharmaceutical containers with at least one solid and sealing them, according to claim 1.

This ionisation prevents the solid dispensed into the container from tending to adhere to the inner walls of the container (1), which may distort the amount of solid dispensed into the container, hinder a visual assessment of the level of product dispensed, or even cause incomplete administration of the product to the patient.

In the case of ionisation in the rod/ring case, re-ionisation will be performed preferably with a needle with or without a gas stream.

In general, throughout the present description, filling is preferably carried out by means of a dispensing needle whose tip or dispensing end is located, throughout the filling stage, at a height of 1 to 3 mm above the surface of the solid deposited on the bottom of the container, in order to prevent generating turbulence that could lift the solid towards the walls of the container.

This procedure has two advantages: On the one hand, achieving accuracy in the filling of substances into a single container even when two or more filling stations are used, by preventing the solid substances from sticking to the sides of the container. On the other hand, ensuring the integrity of the seal of the pharmaceutical container, which is especially important in the case of medicinal products as it prevents both the entry of foreign agents into the container that would contaminate the product, and the leakage of the product to the outside affecting the effective dose of the product.

Additionally, the present invention also solves the problem of static charges generated by collisions between the containers used for filling, whether they are made of glass or polymeric material, in a sterile environment that is generally subject to laminar or turbulent flows which increase the movement and dispersion of electrostatic charges.

Although the invention is generally applicable to powdered solid compounds of any nature, this procedure is particularly applicable to solids having the following particle size distribution:

$$D_{10} \geq 20 \text{ microns}$$

$$70 \text{ microns} \leq D_{50} \leq 110 \text{ microns}$$

$$150 \text{ microns} \leq D_{90} \leq 215 \text{ microns}$$

where $D_{10}$ is the mean value of the particle size that divides the population into exactly two equal halves, with 50% of the distribution being above this value, and 50% below. In general, throughout this specification, a value denoted as "d0.X" or "Dx" represents the mass fraction of the drug with particle sizes below the specified value, having a range of 0.0 to 1.0. According to this definition, a value of d0.1 or $D_{10}$ means that 10% of the total mass of the drug particles has a particle size of 10 microns or less.

This is ideally applicable to solids having the following particle size distribution:

$$D_{10} \geq 25 \text{ microns}$$

$$100 \text{ microns} \leq D_{50} \leq 155 \text{ microns}$$

$$245 \text{ microns} \leq D_{90} \leq 325 \text{ microns}$$

Examples of such compounds are risperidone, paliperidone, fentanyl, olanzcompound activone, letrozole, aripiprazole, anastrozole, asencompound activone, brexiprazole, cariprazine, clozcompound activone, iloperidone, lurasidone, queticompound activone, ziprasidone, among others including any derivatives, metabolites or salts (such as pamoate or palmitate) alone or in combination.

Other examples of such compounds are also biocompatible polymers of the polylactic acid (PLA), polyglycolic acid (PGA) types and their copolymers polylactic co-glycolic acid (PLGA) including any derivatives or copolymers, alone or in combination.

**Brief description of the figures**

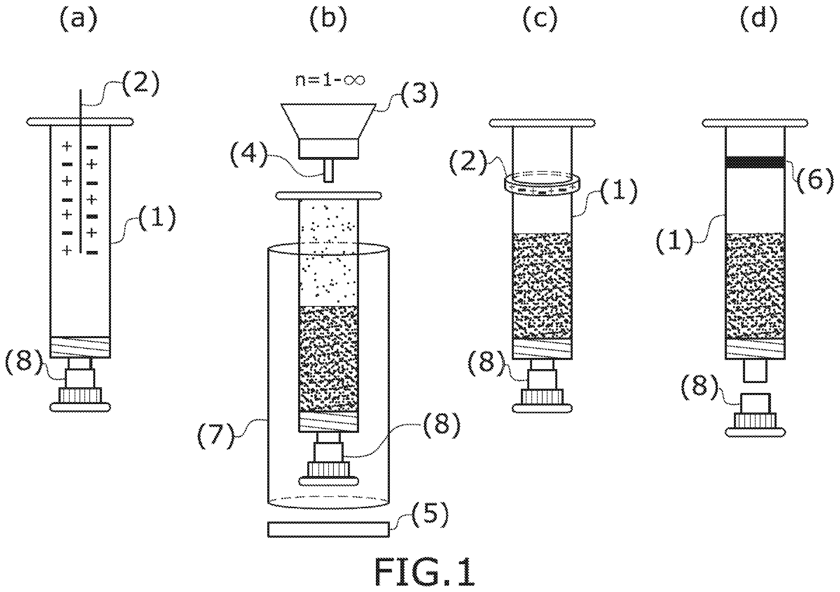

The figures accompanying the present invention serve to illustrate the nature of the invention. These figures are included for purposes of illustration only and should not be understood as limiting the invention claimed herein. With respect to the ionisation phenomenon, the present invention proposes different methods, some of which are shown in figures 1 to 7 described below. In order to properly interpret the figures, the ionisation phenomenon is represented by the alternating positive (+) and negative (-) signs, bearing in mind that this stream of ions of different signs may or may not be accompanied by a stream of sterile carrier gas, although the latter is not represented as such in the figures. The ioniser shown as a ring in the figures is not part of the claimed invention.

Figure 1: Figure 1 shows a particular embodiment of the aseptic filling and sealing process according to the present invention, wherein the pharmaceutical container depicted is, in this case, a male syringe (1) which remains capped with a nozzle cap (8) throughout the process. The syringe (1) is subjected to a first ionisation stage (a) with the aid of an ioniser (2), with either a rod, gun, curtain, blade, barrel, needle or nozzle, or an ionising filter, including isolators with an ioniser on the top of the isolator, although in this case a needle ioniser is shown. The male syringe (1) then passes to the filling station (b), where the procedure of the invention may comprise multiple filling stations, in particular if there are multiple solids to be filled into the syringe. In this station, the syringe, which may optionally be inserted in a cylinder (7), is weighed by a weighing cell (5) during filling, which takes place by means of a hopper (3) and a nozzle or dispensing needle (4). After filling, the male syringe (1) is subjected to another ionisation stage (c) by means of an ioniser (2) provided at this stage either by a rod, gun, curtain, blades, barrels, needle or nozzle, or an ionising filter, among which isolators with an ioniser on the top of the isolator can also be found, although in this case a ring ioniser is shown. Finally, the male syringe (1) is transferred to a sealing station (d) where it is hermetically sealed at the top with a stopper (6).

Figure 2: Shows another particular embodiment of the process of the present invention, where the male syringe of Figure 1 has been replaced by a female syringe as the pharmaceutical container (1).

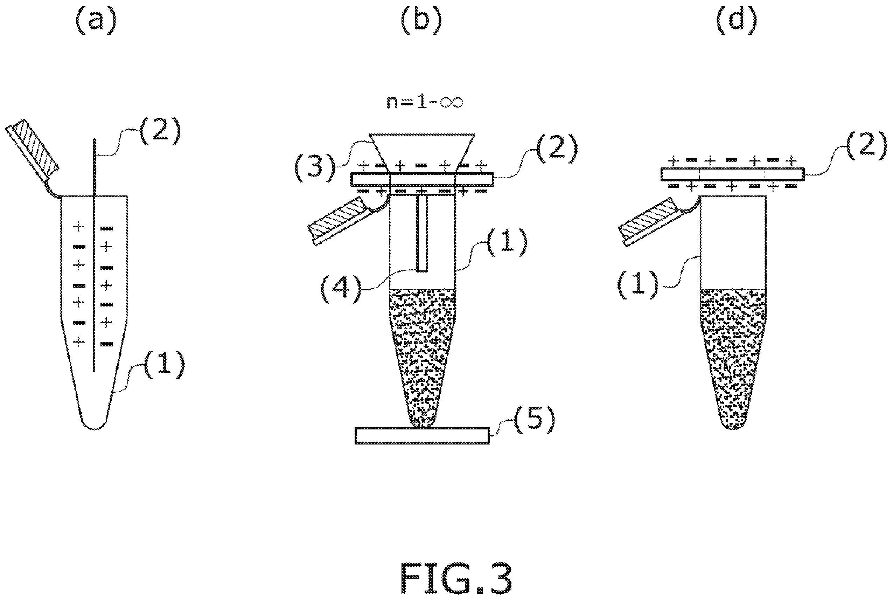

Figure 3: Shows a particular embodiment of the process of the present invention, where the syringe has been replaced as the pharmaceutical container (1) by an Eppendorf® tube, which is subjected to an ionisation stage (a) thanks to the presence in said stage of an ioniser (2) whether it be a rod, gun, curtain, blades, barrels, needle or nozzle, or an ionising filter, among which can also be found isolators with an ioniser on the top of said isolator, in this case a needle ioniser. It is then transferred to the filling station (b) (there may be multiple filling stations) where there is an ioniser (2) with a bar, gun, curtain, blades, barrel, needle or nozzle, or an ionising filter, among which there may also be isolators with an ioniser on the top of the isolator, in this case an ionising filter. In addition to the ioniser, this stage contains a weighing cell (5), a hopper (3), and a nozzle or dispensing needle (4). Finally, after filling, the Eppendorf® tube is subjected to an ionisation process by means of a rod, gun, curtain, blades, barrel, needle or nozzle, or an ionising filter, which may also include isolators with an ioniser on the top of the isolator, in this case a rod ioniser (2).

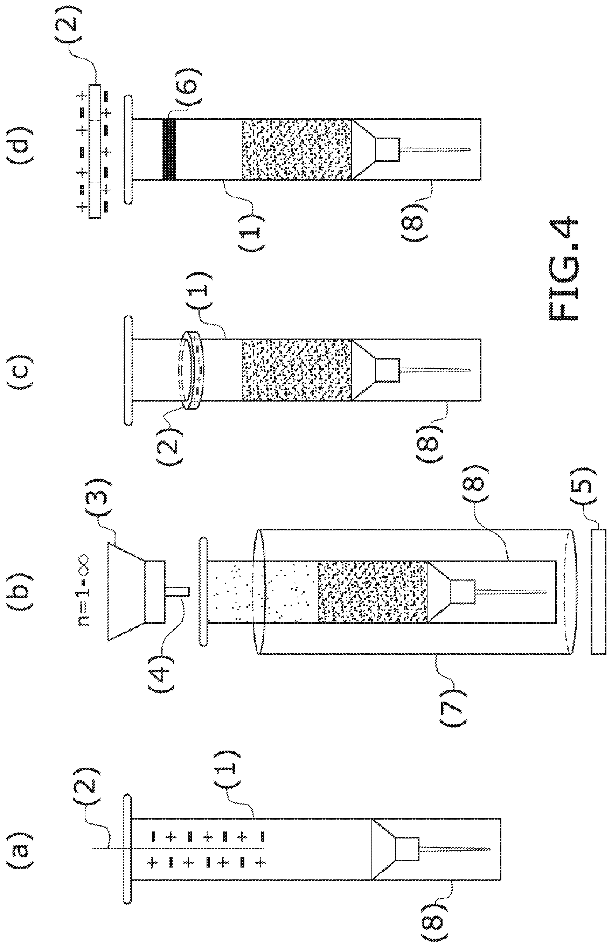

Figure 4: Shows a particular embodiment of the procedure of the present invention in which the container represented is, in this case, a syringe with needle (1) pre-capped with the nozzle cap (8) throughout the sealing process, which is subjected to a first ionisation process (a) with the aid of an ioniser (2) which may be in the form of a bar, gun, curtain, blades, barrel, needle or nozzle, or an ionising filter, among which can also be found isolators with an ioniser on the top of said isolator, although in this case a needle ioniser is shown. The syringe with needle (1) then passes to the filling station (b), where in the procedure described in the present invention there may be multiple filling stations, in particular if there are multiple solids to be filled into the syringe. In said station, the syringe with needle, which may optionally be inserted in a cylinder (7), is weighed by a weighing cell (5) during filling, which takes place by means of a hopper (3) and a nozzle or dispensing needle (4). After filling, the syringe with needle (1) is subjected to a further ionisation step (c) by means of an ioniser (2), with either a rod, gun, curtain, blade, barrel, needle or nozzle, or an ionising filter, including isolators with an ioniser on the top of the isolator, although in this case a ring ioniser is shown. Finally, the syringe with needle (1) is transferred to a sealing station (d) where it is hermetically sealed at the top with a stopper (6), while it is subjected to an additional ionisation stage by means of an ioniser (2) provided at this step, with either a rod, gun, curtain, blades, barrels, needle or nozzle, or an ionising filter, among which isolators with an ioniser on the top of said isolator can also be found, in this case a rod ioniser.

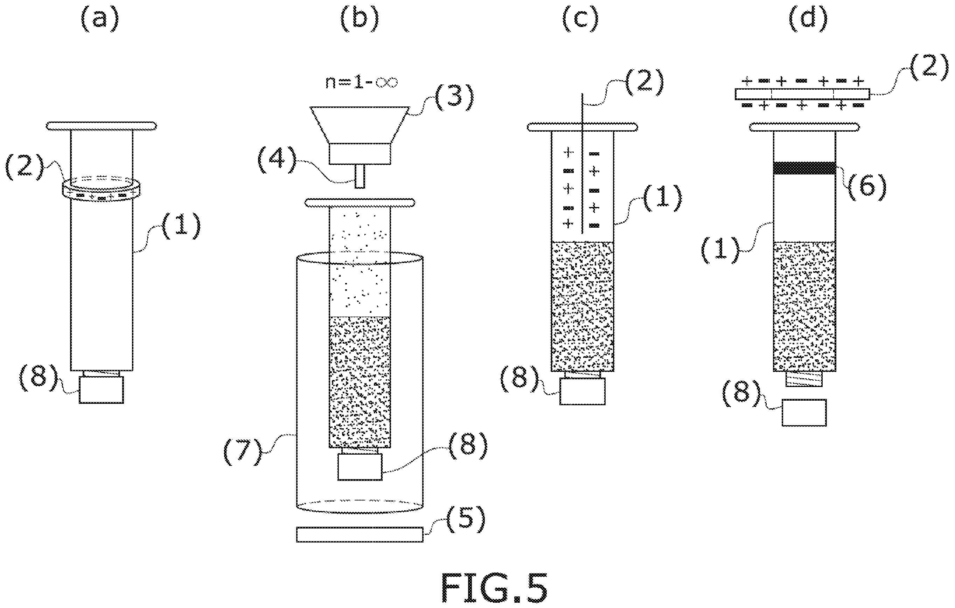

Figure 5: Shows another particular embodiment of the procedure of the present invention in which the container depicted is, in this case, a female syringe (1) capped throughout the process with a nozzle cap (8), which is subjected to a first ionisation process (a) with the aid of an ioniser (2) with either a rod, gun, curtain, blade, barrel, needle or nozzle, or an ionising filter. Next, the female syringe (1) passes to the filling station (b), where in the procedure described in the present invention there may be multiple filling stations, in particular if there are multiple solids to be filled into the syringe. In said station, the syringe, which may optionally be inserted in a cylinder (7), is weighed by a weighing cell (5) during filling, which takes place by means of a hopper (3) and a nozzle or dispensing needle (4). After filling, the syringe undergoes an ionisation process (c) by means of an ioniser (2) in the form of a rod, gun, curtain, blades, barrels, needle or nozzle, or an ionising filter, including isolators with an ioniser on the top of the isolator, although in this case a needle ioniser is shown. Finally, the female syringe (1) is transferred to a sealing station (d) where it is hermetically sealed at the top with a stopper (6) while it is subjected to an additional deionisation phase by means of an ioniser (2), provided at this stage, with either a rod, gun, curtain, blade, barrel, needle or nozzle, or a filter ioniser, among which there may also be isolators with an ioniser on the top of the isolator, in this case, a rod ioniser.

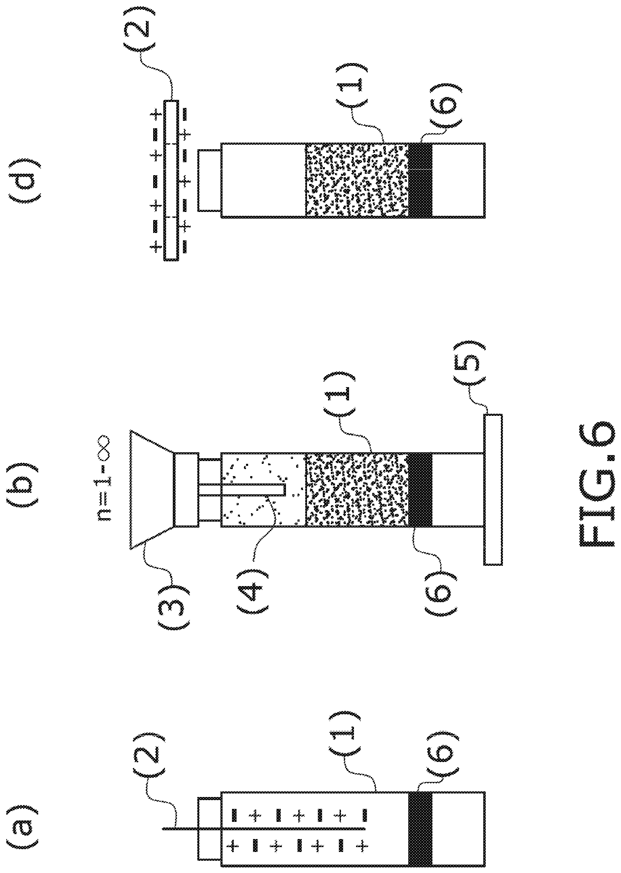

Figure 6: Shows another particular embodiment of the procedure of the present invention in which the container represented is, on this occasion, a cartridge (1), is subjected to a first ionisation process (a) with the aid of an ioniser (2) with either a rod, gun, curtain, blades, barrel, needle or nozzle, or an ionising filter, among which can also be found isolators with an ioniser on the top of said isolator, although in the present case a needle ioniser is represented. The cartridge (1) then passes to the filling station (b), where in the procedure described in the present invention there may be multiple filling stations, particularly if there are multiple solids to be filled into the cartridge. In this station, the cartridge is weighed by a weighing cell (5) during filling, which takes place by means of a hopper (3) and a nozzle or dispensing needle (4). Finally, the cartridge (1), which may optionally be inserted in a cylinder (7), is transferred to an ionisation station where an ioniser (2) will act, with either a rod, gun, curtain, blades, barrel, needle or nozzle, or an ionising filter, or an isolator with an ioniser on the top of the isolator, where in this case a rod ioniser is shown.

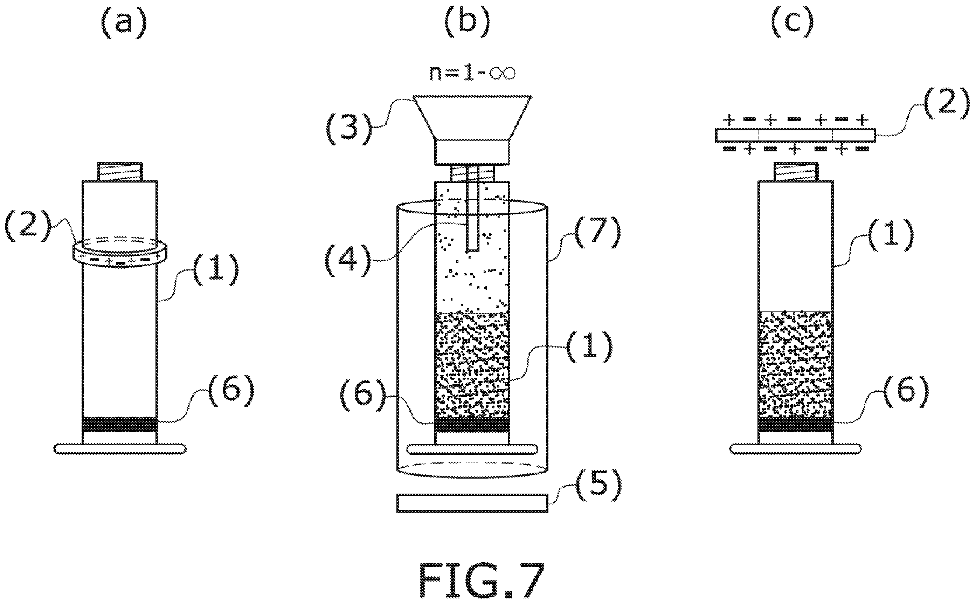

Figure 7: Shows a particular embodiment of the process of the present invention in which the container represented is, in this case, a pre-filled female syringe (1) which is first subjected to an ionisation process (a) with the aid of an ioniser (2) with a rod, gun, curtain, blades, barrels, needle or nozzle, or an ionising filter, among which can also be found isolators with an ioniser on the top of said isolator, although in the present case one with a ring is represented. After this the syringe, which is filled at its threaded end, is located in the filling station (b), where in the process described in the present invention there may be multiple filling stations, particularly if there are multiple solids to be filled into the syringe. In this station, the syringe, which may optionally be inserted in a cylinder (7), is weighed by a weighing cell (5) during filling, which takes place by means of a hopper (3) and a nozzle or dispensing needle (4). After filling, the female syringe (1) is subjected to a further ionisation phase (c) by means of an ioniser (2) with either a rod, gun, curtain, blades, barrels, needle or nozzle, or an ionising filter, which may also include isolators with an ioniser on the top of the isolator (in this case a rod ioniser).

## Detailed description of the invention

Filling of solid substances either by volumetric or gravimetric filling such as powder, granules, pellets, nanoparticles or microparticles in small pharmaceutical containers such as vials, capsules, ampoules, single-dose devices, inhalers, bottles, blister cartridges, sachets, bags, test tubes, Eppendorf® type tubes and syringes (with male or female, threaded or non-threaded nozzles) and of different materials, such as plastics of different composition, such as polyolefins and cyclopolyolefins, polypropylene, polybutadiene, polyethylene, polystyrene, polyvinyl chloride, polyacrylonitrile, polyamides, etc., polyesters (containing the ester functional group in its main chain), poly(ethylene terephthalate), polycarbonate), acrylic polymers (poly(methyl methacrylate), polyacrylonitrile), thermoplastic resins (polyacetals and polyhaloethylenes), polyurethanes, formaldehyde resins (phenol resin, urea resin), phenoplasts, aminoplasts, thioplasts, duroplastic resins (unsaturated polyester, polyurethanes), polyvinylidene silicones, cellulose derivatives, polycarbonates, and mixtures thereof, etc., where also alternatively the container can be made of metal, e.g. steel or titanium suitable for drug administration, or glass, among others, is currently a serious problem for the pharmaceutical industry due to the great drawback of adherence of these substances to the walls of the sealing area of the containers used. This adherence results in significant inconveniences for the cited industry, as it must comply with the regulations indicated in the various international Pharmacopoeias, in addition to complying with good manufacturing practices (GMP). The present invention addresses the drawbacks related to the adhesion of said solid substances to the walls of containers used for filling, as this adhesion hinders both the filling and the aseptic sealing processes. For the aseptic filling and sealing process referred to in the present invention, only solid substances such as those mentioned above are used.

The international Pharmacopoeias require for aseptic filling and sealing the presence of large air flow streams (unidirectional or turbulent regime) to ensure the removal of any extraneous particles that may contaminate the final product. The use of these airflow streams makes filling with solids quite difficult, as a disturbance is generated that causes the solid to adhere to the walls of the container used for filling.

As the solid substances dispensed adhere to the walls of the container sealing area, they are not able to coalesce in the mouth area of the container, thus preventing the necessary sealing from being achieved. This lack of sealing leads to two serious problems: the loss of doses of the solid substance dispensed and the contamination of the container used for filling.

The loss of doses leads to inaccuracy in the administration of the pharmaceutical product, as the solid substances adhering to the walls of the container sealing area will be measured by the weighing cell indicating the precise amount of product to be administered to the patient, but when it is administered to the patient, they will receive a lower dose than indicated, as the solid substances adhering to the sides of the container will not be administered and will remain stuck to the sides of the container.

With regard to the contamination of the container that has been used for filling, this is perhaps the most serious of the drawbacks that can result from the lack of airtightness caused by substance adhering to the walls of the sealing area, as it affects the integrity of the medicinal product and has an impact on the health of the patient receiving the pharmaceutical product. When sealing the container with the stopper, if the walls of the container have solid substances adhered to the sealing area, these substances will remain in this area after the container is sealed, which means that the stopper cannot ensure the integrity of the sealed product, as any kind of substance from the environment could enter the product after the capping stage. Microbial contamination is a very serious issue for pharmaceutical companies, as their products are ideal breeding grounds for micro-organisms such as bacteria, fungi or yeasts. A theoretically sterile but contaminated product can lead to deterioration of the product, loss of the product's potential, pyrogenic reactions after administration to the patient, particularly in parenteral administration, infection and colonisation of micro-organisms in the patient, with the risk of secondary infection. Any micro-organism, whether pathogenic or non-pathogenic, found in a supposedly sterile pharmaceutical product is a hazard.

In view of the significant problems caused by the lack of sealing, the present invention offers a solution to the adherence of solid substances to the sides of the sealing area of the pharmaceutical container by achieving the sealing of said substance. Two methods are used to promote the sealing of the solid substances, namely the control of the height of the dispensing needle and the ionisation of both the pharmaceutical container used for filling and the solid substance to be dispensed, as well as the ionisation of the elements of the dispensing and capping equipment that come into contact with the syringe and/or the powder.

As mentioned in the section on the prior art, there are a number of factors that affect the adhesion of solids to the inner walls of the container, among which is the length of the nozzle. The longer the nozzle and the closer it is to the top level of powder in the container, the less kinetic energy it will have. In addition, the nozzle carries the powder away from the surface of the sealing walls. The inventors of the present invention have found that the ideal distance between the powder and the nozzle tip depends on the dosage, dispensing rate and density of the powder, although typically it is between 1 and 3 mm, more preferably around 2mm. The present invention proposes several options regarding the height of the nozzle:

- The first of these is based on having a nozzle with an exact height (h) of the nozzle measured from the bottom of the container. In this case, the filling process is carried out at the rear of the container, i.e. when the container is a syringe, at the mouth or end with the largest diameter. A minimum height h between the solid to be dispensed and the nozzle must always be kept, specifically 2 mm.

- The second option is to always keep the nozzle at a minimum distance of h = 2 mm from the solid substance being dispensed into the container. This method would mean that the nozzle would not be a fixed element, but instead be mobile and could be raised and lowered as the filling process takes place, always maintaining a distance of 2 mm from the solid substance.

- An alternative to the above could be that the nozzle be provided with a containment element to prevent the powder from dispersing, during filling, above the area being filled.

With regard to the ionisation, the approach relies on the solution for preventing electrostatic charges contained in both the pharmaceutical container to be filled and in the solid substance that will be used to fill it. The container walls and the solid have static charges. If these charges are of opposite signs, the solid will adhere to the inner walls of the container. For this reason, both the container and the solid are ionised.

There are two types of electrostatic charges, negative charges which are electrons of the atoms of the chemical elements, and positive charges which are equivalent to the action of the protons of the atomic nucleus deprived of the electrons of the last shell. Electrons on the surface of an insulating material cannot be easily dissipated unless they have a conductive path to ground, which is why the cylinder is a conductive element as mentioned above. As they cannot circulate easily, they give rise to what is known as static electricity. Electrons are free to move from molecule to molecule in conductors, but protons are inseparable from the atom and cannot move unless the atom itself moves. The amount of electrostatic charge depends on the position or distance relative to each other of the materials in the series and its sign is determined by the propensity of a material to give up or gain electrons, which is what the series actually indicates.

The present invention uses any type of ioniser, such as a rod, gun, curtain, blade, barrel, needle or nozzle, or an ionising filter, including isolators with an ioniser on the top of the isolator. For example, a rod can be used to ionise and neutralise both the environment and the air flow, thus eliminating static charges. The ioniser can be implemented in practice by means of a sterile carrier gas stream, such as compressed air or nitrogen $N_2$, preferably using a nitrogen stream, which has the following functions and/or advantages:

- This nitrogen current serves as a vehicle for displacing the ions generated at the electrodes of the ionisation elements that ionise the surrounding air, producing ions that are carried away by the $N_2$ current. These positive and negative ions are generated by supplying alternating current which, via a transformer, reaches values of up to 8,000 volts with an almost negligible current (4mA). Surfaces treated in this way end up having a neutral charge due to the recombination of charges of different signs and repulsion of charges of like sign.

- Generation of an inert atmosphere inside the containers by displacing the oxygen inside the containers, thus preserving the product from the oxidative effect thereof. The introduction of an inert gas into a vessel, known as inerting, is based on the reduction of the percentage of oxygen below the limiting oxygen concentration (LOC),

- Carrying means in the sweeping effect inside the containers. Alternating ion generation eliminates the static forces that adhere the powder to the container walls. This makes the solid remain in its position without adhering to the container or between the solid particles themselves. Then a slight air flow (0.1-0.8 1/min) performs a sweeping effect with the now disaggregated solid.

With respect to the ionisation phenomenon, the present invention proposes different methods for carrying it out, shown in the accompanying figures 1-7, in which the pharmaceutical container is represented in a non-limiting manner as a male or female syringe, a syringe with needle, a cartridge or carpule, or an Eppendorf® tube. Ionisers can be used with any type of ioniser (whether or not accompanied by a stream of sterile carrier gas), such as a rod, gun, curtain, blade, barrel, needle or nozzle, or an ionising filter, including isolators with an ioniser on the top of the isolator, such as a rod, to ionise and neutralise both the environment and the airflow, thus eliminating static charges. In the case of isolators, and according to a preferred embodiment, prior to the dosing operation described in the present invention, sterilisation with nebulised or vaporised hydrogen peroxide or a mixture of hydrogen peroxide with peracetic acid is required.

When the pharmaceutical container is removed from the tray, it has a very high electrostatic charge (more than 30,000 Volts). This is due to the constant friction between the container and the tray. That is why, as shown in the various figures, both before the container is inserted into the cylinder and after it has been inserted, the containers are preferably exposed to an ioniser of any type and to a stream of sterile carrier gas. This gas can be nitrogen, which carries ionised air molecules, or compressed air, which hits the inside of the container as well as the sealing area in order to suppress the electrostatic charge they have.

Figure 1 shows a general procedure for aseptic filling of a container comprising several steps:
The container (1), optionally inserted in a supporting cylinder (7) and capped with a nozzle cap (8), is subjected to an ionisation stage (a) by means of an ioniser (2), of any type, e.g. rod, gun, curtain, blades, barrel, needle or nozzle, or an ionising filter, which may also include isolators with an ioniser on the top of the isolator, or any other type of ioniser. The ioniser (2) is used to remove the electrostatic charge from the container both on the inside walls and in the sealing area. This ioniser may or may not be applied together with a sterile stream of a carrier gas such as nitrogen, carrying ionised air molecules, or compressed air carrying ionised air molecules, although more preferably a nitrogen stream carrying ionised air molecules is used. The nitrogen stream carrying ionised air molecules that is used reaches both the inside of the container and the sealing area. By these two ionisation processes (ionisation with ioniser and the optional application of a sterile gas stream), the electrostatic charges in the container are removed so that the container can be filled.

After the ionisation process (a), the syringe (1) passes to the aseptic filling station (b). In this stage the aseptic filling of the container (1) with the solid substance is carried out. This process requires the use of a hopper (3) containing the solid substance to be dispensed and a dispensing needle or nozzle (4) through which the solid substance is dispensed. A weighing cell (5) is also required to measure accurately the amount of solid substance that is dispensed. This station may or may not have a stream of a sterile carrier gas, such as compressed air or nitrogen, that carries ionised air molecules, preferably nitrogen carrying ionised air molecules to provide a vehicle for the ions. There are as many filling stations as products or combinations thereof will be filled.

After filling the container (1) with the solid, it is subjected to a deionisation stage (c) by means of a deioniser (2), which may be a rod, gun, curtain, blades, barrels, needle or nozzle, or an ionising filter, including isolators with an ioniser on the top of the isolator. Together with the ioniser, a stream of a sterile carrier gas such as compressed air or nitrogen carrying ionised air molecules may or may not be applied; preferably, nitrogen carrying ionised air molecules is used to prevent the solid from adhering to the walls of the sealing area of the container (1).

Finally, there is the aseptic sealing station (d), where the stopper (6) is inserted to seal the container. The latter station may or may not use a stream of a sterile carrier gas such as compressed air or nitrogen, preferably carrying ionised air molecules.

Figure 2 shows another embodiment of the ionisation process consisting of four stages:
The first stage consists in an ionisation process (a) similar to that shown in figure 1, in which a stream of a sterile carrier gas, such as nitrogen carrying ionised air molecules or compressed air carrying ionised air molecules, may or may not be introduced into the container (1), the syringe having been previously capped with the nozzle cap (8) and optionally introduced into the carrier cylinder (7). This sterile carrier gas stream is used in conjunction with an ioniser (2), whether in the form of a rod, gun, curtain, blade, barrel, needle or nozzle, or an ionising filter, which may also include isolators with an ioniser on the top of the isolator. The stream and ioniser (2) reach both the bottom of the container (1) and the sealing zone;

in the present figure, the sterile carrier gas stream used is preferably a nitrogen stream carrying ionised air molecules. After applying this stream, the container is free of electrostatic charges in order to start filling.

In the second stage of the described process, the container (1) passes to the aseptic filling station (b) where aseptic filling with the solid is carried out. In this station there are several elements such as: a hopper (3) in which the solid substance to be dispensed is located, a dispensing needle or nozzle (4) in charge of dispensing the solid, and a weighing cell (5) to control the exact amount of solid that is dispensed. There are as many filling stations as products or combinations thereof will be filled. In order to ensure the cleanliness of the sealing zone, a third stage is used which consists in an ionisation stage (c) where an ioniser (2) is used, which may be a rod, gun, curtain, blade, barrel, needle or nozzle, or an ionising filter, among which there may also be isolators with an ioniser on the top of the isolator, where a stream of sterile carrier gas such as nitrogen carrying ionised air molecules or compressed air may or may not be applied as well in order to break the adhesion of the solid to the sides of the container in the sealing zone. Preferably, a nitrogen stream carrying ionised air molecules is used to serve as a vehicle for ion displacement and as a carrier means in the sweeping effect, thus obtaining the desired sealing phenomenon in this station. There will be different numbers of ionisation stations according to the needs of each product.

Finally, the last stage consists in a sealing stage (d) in which the container is sealed with a stopper (6). A sterile carrier gas stream such as nitrogen, preferably carrying ionised air molecules, may or may not be used at this stage, because although the sealing area is clean of solids, it is necessary to ensure that the container is completely clean and that none of the dispensed solids adhere to the stopper used for sealing and to the container walls due to the electrostatic charges created by the friction when the container is placed in the sealer.

Figure 3 shows another particular realisation of the ionisation process, comprising the following steps:
The container (1) is subjected to an ionisation process (a), by means of an ioniser (2) which can be a rod, gun, curtain, blade, barrel, needle or nozzle, or an ionising filter, including isolators with an ioniser on the top of the isolator. This ioniser may or may not be applied together with a sterile carrier gas stream such as nitrogen carrying ionised air molecules or compressed air carrying ionised air molecules, although more preferably a nitrogen stream carrying ionised air molecules is used. The ioniser (2) is used to remove the electrostatic charge from the container both on the inside walls and in the sealing area. In addition, the nitrogen stream carrying ionised air molecules used reaches both the inside of the container and the sealing area; with these two ionisation processes, the electrostatic charges on the container are eliminated so that it can then be filled.

Subsequently, after this ionisation phase (a), the container (1) passes to the aseptic filling station (b) where it is aseptically filled with the solid. In this station there are several elements such as a hopper (3) where the solid substance to be dispensed is located, a dispensing needle or nozzle (4) in charge of dispensing the solid, a weighing cell (5) to control the exact amount of solid that is dispensed and an ioniser (2) which can be a bar, gun, curtain, blades, barrel, needle or nozzle, or an ionising filter, among which there can also be isolators with an ioniser on the top of the isolator. This station may or may not have a stream of a sterile carrier gas, such as compressed air or nitrogen, that carries ionised air molecules, preferably nitrogen carrying ionised air molecules to provide a vehicle for the ions. This ensures that the dispensed solid does not remain in the sealing zone. There will be as many filling stations as there are products or combinations thereof to be filled, as well as as many ionisation stages as necessary.

After the container (1) has been filled with the solid in the filling station (b), in order to ensure the cleanliness of the sealing area of the container the last stage is carried out, which is an ionisation process (c) using an ioniser (2) either in the form of a rod, gun, curtain, blades, barrel, needle or nozzle, or an ionising filter, including isolators with an ioniser on the top of the isolator which may or may not act in conjunction with a stream of sterile carrier gas such as nitrogen carrying ionised air molecules or compressed air in order to break the adhesion of the solid to the sides of the container in the sealing zone. Preferably, a nitrogen stream carrying ionised air molecules is used to serve as a vehicle for ion displacement and as a carrier means in the sweeping effect, thus obtaining the desired sealing phenomenon in this station. There will be different numbers of ionisation stations according to the needs of each product.

Figure 4 shows another particular embodiment of the procedure described in the present invention:
The container (1), optionally inserted in a supporting cylinder (7) and capped with the nozzle cap (8), is subjected to an ionisation process (a) by means of an ioniser (2), of any type in the form of a rod, gun, curtain, blades, barrel, needle or nozzle, or an ionising filter, which may also include isolators with an ioniser on the top of the isolator, or any other type of ioniser. This ioniser may or may not be applied together with a sterile carrier gas stream such as nitrogen carrying ionised air molecules or compressed air carrying ionised air molecules, although more preferably a nitrogen stream carrying ionised air molecules is used. The ioniser (2) is used to remove the electrostatic charge from the container both on the inside walls and in the sealing area. In addition, the nitrogen stream carrying ionised air molecules used reaches both the inside of the container and the sealing area; with these two ionisation processes, the electrostatic charges on the container are eliminated so that it can then be filled.

After this ionisation phase (a), the container (1) passes to the aseptic filling station (b) where it is aseptically filled with the solid. This process requires the use of a hopper (3) containing the solid substance to be dispensed and a dispensing needle or nozzle (4) through which the solid substance is dispensed. A weighing cell (5) is also required to measure accurately the amount of solid substance that is dispensed. This station may or may not have a stream of a sterile carrier gas, such as compressed air or nitrogen, that carries ionised air molecules, preferably nitrogen carrying ionised air molecules to provide a vehicle for the ions. There are as many filling stations as products or combinations thereof will be filled.

After the container (1) has been filled with the solid in the filling station (b), in order to ensure that the sealing area of the container is clean, a third stage is carried out, which is an ionisation process (c) in which an ioniser (2) is used, either in the form of a rod, gun, curtain, blades, barrel, needle or nozzle, or an ionising filter, among which isolators with an ioniser on the top of the isolator can also be found. This ioniser may or may not act together with a stream of a sterile carrier gas such as nitrogen carrying ionised air molecules or compressed air, in order to break the adhesion of the solid to the sides of the container in the sealing area. Preferably, a nitrogen stream carrying ionised air molecules is used to serve as a vehicle for ion displacement and as a carrier means in the sweeping effect, thus obtaining the desired sealing phenomenon in this station. There will be different numbers of ionisation stations according to the needs of each product.

Finally, the container (1) passes to the sealing station (d) where it is sealed with a stopper (6). In this stage an ioniser (2) is used which may be in the form of a rod, gun, curtain, blade, barrel, needle or nozzle, or an ionising filter, among which there may also be isolators with an ioniser on the top of the isolator which may or may not act together with a stream of sterile carrier gas such as nitrogen, preferably carrying ionised air molecules, because, although the sealing area is clean of solid substances, it is necessary to ensure that the container is completely clean and that none of the dispensed solid substances adheres to the stopper used for sealing and to the walls of the container due to the electrostatic charges created by friction when the container is placed in the sealing machine.

Figure 5 shows another particular embodiment of the procedure described in the present invention.

The container (1) which has been capped with the nozzle cap (8) and optionally inserted into a support cylinder (7) is subjected to an ionisation process (a) by means of an ioniser (2), whether in the form of a rod, gun, curtain, blade, barrel, needle or nozzle, or an ionising filter, including isolators with an ioniser on the top of the isolator, which may or may not be in conjunction with a sterile carrier gas stream such as nitrogen carrying ionised air molecules or compressed air carrying ionised air molecules, but more preferably nitrogen carrying ionised air molecules. The ioniser (2) is used to remove the electrostatic charge from the container both on the inside walls and in the sealing area. In addition, the nitrogen stream carrying ionised air molecules used reaches both the inside of the container and the sealing area; with these two ionisation processes, the electrostatic charges on the container are eliminated so that it can then be filled.

After this ionisation process (a), the container (1) passes to the aseptic filling station (b) where it is aseptically filled with the solid. In this station it is necessary to use several elements such as a hopper (3) where the solid substance to be dispensed is located, a dispensing needle or nozzle (4) in charge of dispensing the solid, and a weighing cell (5) to control the exact amount of solid that is dispensed. This station may or may not have a stream of a sterile carrier gas, such as compressed air or nitrogen, that carries ionised air molecules, preferably nitrogen carrying ionised air molecules to provide a vehicle for the ions. This ensures that the dispensed solid does not remain in the sealing zone. There are as many filling stations as products or combinations thereof will be filled.

After this filling stage (b), the container is subjected to an ionisation stage (c) in which the ioniser (2) may be in the form of a rod, gun, curtain, blades, barrel, needle or nozzle, or an ionising filter, which may also include isolators with an ioniser on the top of the isolator, which may or may not be in conjunction with a sterile carrier gas stream such as nitrogen carrying ionised air molecules or compressed air carrying ionised air molecules, although more preferably the nitrogen stream carrying ionised air molecules is used.

Finally, the container (1) passes to the sealing station (d) where it is sealed with a stopper (6). In this stage an ioniser (2) is also used which may be in the form of a rod, gun, curtain, blade, barrel, needle or nozzle, or an ionising filter, among which there may also be isolators with an ioniser on the top of the isolator which may or may not act together with a stream of sterile carrier gas such as compressed air or preferably nitrogen carrying ionised air molecules, because, although the sealing area is clean of solid substances, it is necessary to ensure that the container is completely clean and that none of the dispensed solid substances adheres to the stopper used for sealing and to the walls of the container due to the electrostatic charges created by friction when the container is placed in the sealing machine.

Figure 6 shows three stages:

In the first stage the container (1) is subjected to an ionisation process (a) by means of an ioniser (2), whether in the form of a rod, gun, curtain, blade, barrel, needle or nozzle, or an ionising filter, including isolators with an ioniser on the top of the isolator, which may or may not be in conjunction with a sterile carrier gas stream such as nitrogen carrying ionised air molecules or compressed air carrying ionised air molecules, although more preferably nitrogen carrying ionised air molecules is used. The ioniser (2) is used to remove the electrostatic charge from the container both on the inside walls and in the sealing area. In addition, the nitrogen stream carrying ionised air molecules used reaches both the inside of the container and the sealing area; with these two ionisation processes, the electrostatic charges on the container are eliminated so that it can then be filled.

After this ionisation process (a), the container (1) passes to the aseptic filling station (b) where it is aseptically filled with the solid. This process requires the use of a hopper (3) containing the solid substance to be dispensed and a dispensing needle or nozzle (4) through which the solid substance is dispensed. A weighing cell (5) is also required to measure accurately the amount of solid substance that is dispensed. This station may or may not have a stream of a sterile carrier gas, such as compressed air or nitrogen, that carries ionised air molecules, preferably nitrogen carrying ionised air molecules to provide a vehicle for the ions. There are as many filling stations as products or combinations thereof will be filled.

Lastly, the container (1) passes to the ionisation station. In this stage an ioniser (2) is used which may be in the form of a rod, gun, curtain, blade, barrel, needle or nozzle, or an ionising filter, among which there may also be isolators with an ioniser on the top of the isolator which may or may not act together with a stream of sterile carrier gas such as nitrogen, preferably carrying ionised air molecules, because, although the sealing area is clean of solid substances, it is necessary to ensure that the container is completely clean and that none of the dispensed solid substances adheres to the stopper and to the walls of the container due to the electrostatic charges created by friction when the container is placed in the sealing machine.

Figure 7 shows a particular embodiment of the procedure described in the present invention:

The container (1) already capped with a stopper (6) and optionally inserted in a cylinder (7) is ionised in an ionisation stage (a) by means of an ioniser (2) in the form of a rod, gun, curtain, blade, barrel, needle or nozzle, or an ionising filter, among which can also be isolaters with an ioniser on the top of said isolator. This station may or may not have a stream of a sterile carrier gas, such as compressed air or nitrogen, that carries ionised air molecules, preferably nitrogen carrying ionised air molecules to provide a vehicle for the ions.

The container is then placed in the aseptic filling station (b), where it is aseptically filled with the desired solid with the nozzle part. In this station it is necessary to use several elements, such as a hopper (3) where the solid substance to be dispensed is located, a dispensing needle or nozzle (4) in charge of dispensing the solid, and a weighing cell (5) to control the exact amount of solid that is dispensed. There may or may not be a stream of a sterile carrier gas, such as compressed air or nitrogen, that carries ionised air molecules, preferably nitrogen carrying ionised air molecules to provide a vehicle for the ions. This ensures that the dispensed solid does not remain in the sealing zone. There are as many filling stations as products or combinations thereof will be filled.

After the container (1) has been filled with the solid in the filling station (b), in order to ensure that the sealing area of the container is clean, a final stage is carried out, which is an ionisation process (c) in which an ioniser (2) is used, either in the form of a rod, gun, curtain, blades, barrel, needle or nozzle, or an ionising filter, among which isolators with an ioniser on the top of the isolator can also be found. This ioniser may or may not be next to a stream of a sterile carrier gas such as nitrogen carrying ionised air molecules or compressed air, in order to break the adhesion of the solid to the sides of the container in the sealing area. Preferably, a nitrogen stream carrying ionised air molecules is used to serve as a vehicle for ion displacement and as a carrier means in the sweeping effect, thus obtaining the desired sealing phenomenon in this station. There will be different numbers of ionisation stations according to the needs of each product.

In the preferred embodiments shown in the figures, an additional ionisation stage of the container is executed after filling with the solid substance and immediately prior to sealing with the stopper. Preferably, the ionisation must be performed also when the container is empty, before it is filled with the solid substance, and more preferably every station of the filling and sealing station must include ionisers to ionise both the container and the solid substance, thereby preventing adherence of the latter to the inner walls and ensuring the cleanliness of the container. These ionisers may be of any type, such as rod, gun, curtain, blade, barrel, needle or nozzle, or an ionising filter, among which can also be isolators with an ioniser on the top of said isolator. To aid the dosing, a stream can be used of a sterile carrier gas such as compressed air or nitrogen, carrying ionised air molecules, as it provides a vehicle for displacement of the ions, generates an inert atmosphere, and provides a carrier means in the sweeping effect. This nitrogen current carrying ionised air molecules is used in the ionisation stations together with an ioniser. More preferably, it is used in all the stations of the aseptic filling and sealing process.

**Examples**

The following specific examples are provided to illustrate the nature of the present invention. These examples are included for purposes of illustration only and should not be understood as limiting the invention claimed herein.

These examples use as containers cartridges or carpules, syringes with a needle or with a catheter cone, Luer cone, or Luer lock cone; all of them with a male tip or female tip and Eppendorf® tubes, as excipients biocompatible polymers of the PLGA (lactic or glycolic acid) and PLA (poly(lactic acid)) type, and as active ingredients Risperidone and Letrozole respectively.

Example 1: Filling of a 50 mg dose of Risperidone in a syringe with a male nozzle or male syringe.

In this example, two products must be filled in a pharmaceutical container, specifically in a glass syringe with a male nozzle that has been precapped with the nozzle cap (8). The products to be filled are the excipient PLA and the active ingredient Letrozol, specifically a 50 mg dose. It should be noted that the filling process takes place inside a rigid-walled aseptic isolator. Before starting the filling process, all the equipment must be clean and sterile. For this purpose the equipment is previously sterilised with nebulised or vaporised hydrogen peroxide, or with a mixture of hydrogen peroxide and peracetic acid.

The isolator comprises two main sections: (i) the transfer chamber (TC), which is a chamber that facilitates loading sterile materials to and from the working chamber of the isolator, as all the materials and tools loaded in the sterile isolator must be previously sterilised; and (ii) the working chamber (MC), which contains filling equipment for the excipient and the active ingredient, and a syringe capping or sealing unit.

To start the filling the male syringes (1) and the caps (6) are taken, both sterile, delivering said caps to the operator in the capping or sealing station (d) so that they are inserted in the syringe sealing machine. There are as many filling stations as products or combinations thereof will be filled.

Both the PLA used as excipient and the Letrozole used as active ingredient are delivered to the operators at the filling station (b), who load them in their respective hoppers (3). The male syringes (1) that will be used for filling undergo an ionisation process from the rear part of the syringes or from the collar of the syringes using a needle ioniser (2). In this way the male syringes (1) are ionised (a) to eliminate the electrostatic charge inside them and in the sealing area of the syringe body. The male syringe (1) is then placed upside down inside a cylinder (7).

The cylinder (7) containing the ionised male syringe (1) is directed towards the filling station (b) to fill with PLA. The male syringe (1) is placed in the weighing cell (5), taring its weight to zero. After this, the male syringe (1) is filled at the end proximal to the collar with 90 mg $\pm$ 30% of PLA. Filling is carried out with a dispensing needle or nozzle (4), both made from a non-conducting material. The male syringe (1) is continuously weighed during filling so that the system can be controlled to stop filling when the desired weight is precisely reached, in this case between 90 mg $\pm$ 30%.

After this process, the electrostatic charge inside the inner walls of the container (1) is measured and the measurement obtained is 1075 volts.

After the filling station (b) with the PLA excipient, the male syringe (1) is subjected to an ionisation process (c) using a ring ioniser located on the outside of the syringe to facilitate sealing by preventing the PLA from adhering to the walls of the male syringe (1). During this process, the presence of a stream of nitrogen or sterile carrier gas with ionised air molecules is necessary to displace the ions and act as a carrier for the powder sweeping effect.

After this process, the electrostatic charge inside the inner walls of the container (1) is measured and the measurement obtained is 775 volts.

Once the excipient has been filled and ionised, the cylinder (7) with the male syringe (1) filled with PLA is placed in the filling station (b) in order to fill it with 50 mg ± 30% of Letrozole. The cylinder with the male syringe (1) is placed in the weighing cell (5) where it is tared before filling with the active product. The male syringe (1) is continuously weighed during filling so that filling can be stopped once the desired weight has been reached.

After this filling process of the male syringe (1), it is subjected to another ionisation process (c) with the help of a ring ioniser (2) to prevent both the excipient and the active ingredient with which the male syringe (1) has been filled from adhering to the walls of the male syringe (1). This process requires the presence of a stream of nitrogen or sterile carrier gas with ionised air molecules to displace the ions and act as a carrier for the powder sweeping effect.

After this process, the electrostatic charge inside the inner walls of the container (1) is measured and the measurement obtained is 645 volts.

After filling with the active ingredient and subsequent ionisation, the cylinder (7) with the male syringe (1) passes to the sealing station (d) where the stopper (6) is fitted. This process requires the presence of a stream of nitrogen or sterile carrier gas carrying ionised air molecules to displace the ions and act as a carrier.

After this process, the electrostatic charge inside the inner walls of the container (1) is measured and the measurement obtained is 375 volts.

In this way the desired sealing phenomenon is achieved and the cleanliness of the sealing area inside the syringe body at the distal end of the nozzle thereof is ensured. It also prevents both PLA and Letrozole from sticking to the stopper (6) used for sealing and to the walls of the container due to the electrostatic charges created by the friction created when the container is placed in the sealer.

Once the male syringe has been filled and sealed, it can be placed on a tray with the rest of the filled and sealed syringes.

Example 2: Filling of a 400 mg dose of Letrozole in a syringe with a female nozzle or female syringe of plastic material.

In this second example, PLA is also used as excipient and Letrozole as active ingredient for a dose of 400 mg, and the filling process also takes place inside a rigid-walled aseptic isolator in the same way as in example 1.

Both the PLA used as excipient and the Letrozole used as active ingredient are delivered to the operators at the filling station (b), who load them into their respective hoppers (3), in this case without the hoppers being made of insulating material. The female syringes (1), pre-capped with the nozzle cap (8), that will be used for filling are arranged under a stream of nitrogen or sterile carrier gas carrying ionised air molecules; a needle ioniser (2) is added to this process, such that the female syringes are ionised (a) to eliminate the electrostatic charge inside them and in the sealing zone.

The cylinder (7) containing the ionised female syringe (1) is directed towards the filling station (b) with PLA. The female syringe (1) is placed in the weighing cell (5), taring its weight to zero. After this, the syringe (1) is filled from the back of the syringes or from the collar part of the syringes with an amount of 500 mg + 30% PLA, this filling is done by means of a nozzle (4) or dispensing needle, which is not made of insulating material. The syringe (1) is continuously weighed during filling, so that the system can be controlled to stop filling when the desired weight, in this case between 500 mg + 30%, is reached. During this process the presence of a stream of nitrogen or sterile carrier gas with ionised air molecules is required to displace the ions and provide a carrier in the sweeping effect.

After this process, the electrostatic charge inside the inner walls of the container (1) is measured and the measurement obtained is 1770 volts.

Subsequently, the cylinder (7) with the female syringe (1) filled with PLA undergoes an ionisation process (c) before being filled in a second filling station (b) with the active ingredient Letrozole. For the ionisation, a ring ioniser (2) is used to ionise the PLA adhering to the walls of the sealing area of the syringe (1). A stream of nitrogen or sterile carrier gas carrying ionised air molecules is also used to serve as a vehicle for the displacement of the ions and as a carrier in the sweeping effect, thus achieving the desired sealing phenomenon in this ionisation process.

After this process, the electrostatic charge inside the inner walls of the container (1) is measured and the measurement obtained is 975 volts.

After ionisation, the cylinder (7) with the female syringe (1) filled with PLA is placed in the filling station (b) with 400 mg ± 30% of the active ingredient Letrozole. The cylinder (7) with the female syringe (1) is placed in the weighing cell (5) where it is tared before filling with the active ingredient, and then filling with Letrozole begins. The syringe (1) is continuously weighed during filling so that filling can be stopped once the desired weight has been reached.

It is then transferred to an ionisation station (c) in which a ring ioniser (2) is used to prevent adherence of both the excipient and the active substance to its walls.

After this process, the electrostatic charge inside the inner walls of the container (1) is measured and the measurement obtained is 895 volts, as both the dispensing needle and the hopper are not insulating materials.

After filling with the active ingredient and subsequent ionisation, the cylinder (7) with the female syringe (1) (d) is filled with the active ingredient and a stopper (6) is fitted in it. This process requires the presence of a stream of nitrogen or sterile carrier gas carrying ionised air molecules to displace the ions and act as a carrier for the powder. By this means, the desired sealing phenomenon is achieved and the cleaning of the sealing area inside the syringe body is ensured to leave a clean area in which the stopper is inserted. Furthermore, it prevents both PLA and Letrozole from adhering to the stopper (6) used for sealing and to the walls of the container due to the electrostatic charges created by friction when the container is placed in the sealer.

At this point, the electrostatic charge inside the inner walls of the container (1) is measured and the measurement obtained is 495 volts.

Once the syringe filling process is completed and it has been sealed, the syringe can be placed on a tray with the rest of the filled and sealed syringes.

Example 3: Filling of a 50 mg dose of Letrozole in Eppendorf® tubes.

In this example PLA is used as excipient and Letrozole as active ingredient, for a dose of 50 mg. It should be noted that the filling process takes place inside a rigid-walled aseptic isolator, as in the previous examples.

Both the PLA used as excipient and the Letrozole used as active ingredient are delivered to the operators at the filling station (b), who load them in their respective hoppers (3). The Eppendorf® tubes (1) to be used for filling are arranged under a stream of nitrogen or sterile carrier gas carrying ionised air molecules; a needle ioniser (2) is added to this process and the Eppendorf® tubes (1) are ionised to remove the electrostatic charge inside them and in the sealing zone.

At this point the electrostatic charge inside the inner walls of the container (1) is measured and the measurement obtained is 695 volts, as the tube is made of insulating material and the dimensions of the tube are significantly different from the syringes, as they are wider and shorter than the syringes of examples 1 and 2.

The ionised Eppendorf® tube (1) is led to the filling station with 50 mg ± 30% Letrozole. The Eppendorf® tube (1) is placed in the weighing cell (5), taring its weight to zero. The Eppendorf® tube (1) is then filled with the active ingredient by means of a nozzle (4) made of insulating material. The Eppendorf® tube (1) is continuously weighed during filling, so that the system can be controlled to stop filling when the desired weight is reached. While filling with Letrozole, it is necessary to use an ionisation filter (2) to prevent it from sticking to the walls of the sealing area.

After the filling stage (b), the Eppendorf® tube (1) is again subjected to an ionisation process (c) with the aid of a rod ioniser (2), to ensure that no active ingredient remains adhering to the walls of the container (1).

Again, the electrostatic charge inside the inner walls of the container (1) is measured and the measurement obtained is 700 volts.

After filling with the active ingredient, the Eppendorf® tube (1) filled with Letrozole is placed in the second filling station (b), this time with 90 ± 30% of the excipient PLA. The Eppendorf® tube (1) is placed in the weighing cell (5) where it is tared before filling with the excipient, after which filling with PLA begins. The Eppendorf® tube (1) is continuously weighed during filling, so that filling can be stopped once the desired weight has been reached. During this process, the use of an ionisation filter (2) is necessary to prevent both Letrozole and PLA from adhering to the sealing area.

After this filling process of the Eppendorf® tube (1), the tube is subjected to another ionisation process (c) with the aid of a rod ioniser (2) to prevent both the excipient and the active ingredient with which the Eppendorf® tube (1) has been filled from adhering to the walls of the container (1). The presence of a stream of nitrogen or sterile carrier gas carrying ionised air molecules is also necessary to displace ions and act as a carrier. These two means ensure that the electrostatic charge on the inside of the inner walls of the container (1) is about 595 volts, thus achieving the desired sealing phenomenon and ensuring the cleanliness of the sealing area, as well as preventing both PLA and Letrozole from adhering to the stopper (6) used for sealing and to the walls of the container due to the electrostatic charges created by friction when the container is placed in the sealing machine.

Example 4. Filling of a 75 mg dose of Risperidone in a syringe with needle or syringe with needle made of plastic material.

In this example, PLGA is used as excipient and Risperidone as active ingredient, for a dose of 75 mg. The filling process also takes place inside a rigid-walled aseptic isolator using the same material sterilisation operation as in the previous examples.

The syringes with needle (1) to be used for filling are closed with the nozzle cap (8) and are subjected to an ionisation process with a needle ioniser (2), thereby ionising (a) the syringes (1) in order to eliminate the electrostatic charge inside them and in the sealing area. The cylinder (7) containing the syringe with the ionised needle (1) is made in this case of an insulating material. The syringe (1) is placed in the weighing cell (5), taring its weight to zero. After this, the male syringe (1) is filled with 75 mg ± 30% of PLGA by means of a nozzle (4) made of insulating material. The syringe (1) is continuously weighed during filling so that the system can be controlled to stop filling when the desired weight, in this case between 75 mg ± 30%, is reached.

After this process, the electrostatic charge inside the inner walls of the container (1) is measured and the measurement obtained is 775 volts.

After filling the needle syringe (1) with the excipient, it is subjected to an ionisation process (c) by means of a ring ioniser (2), thus preventing the PLGA from adhering to the walls of the sealing area of the container (1).

After this process, the electrostatic charge inside the inner walls of the container (1) is measured and the measurement obtained is 550 volts.

After filling with the excipient and subsequent ionisation, the cylinder (7) with the syringe (1) filled with PLGA is placed in the next filling station (b) with the active ingredient Risperidone. The cylinder (7) with the syringe (1) is placed in the weighing cell (5), where it is tared before filling with the active ingredient, and then filling with Risperidone begins. The syringe (1) is continuously weighed during filling so that filling can be stopped once the desired weight has been reached.

After filling with the active ingredient, the cylinder (7) with the needle syringe (1) is again subjected to an ionisation process, where with the aid of a ring ioniser (2), both the active ingredient and the excipient are prevented from adhering to the walls of the sealing area of the male syringe (1). After this process, the electrostatic charge inside the inner walls of the container (1) is measured and the measurement obtained is 470 volts.

After this ionisation process, the male syringe (1) passes to the sealing station (d) where it is fitted with a stopper (6) and undergoes a further ionisation process. This process requires a rod ioniser (2) to ionise the PLGA and Risperidone that are adhered to the walls of the sealing area.

After this process, the electrostatic charge inside the inner walls of the container (1) is measured and the measurement obtained is 199 volts.

The ionisation process ensures an optimum sealing and the total cleanliness of the sealing area, as well as preventing both PLGA and Risperidone from adhering to the stopper (6) used for sealing and to the walls of the container due to the electrostatic charges created by the friction created when the container is placed in the sealer.

Example 5: Filling of a 100 mg dose of Risperidone in a syringe with a female nozzle or a female syringe made of plastic material.

In this example, PLGA is used as excipient and Risperidone as active ingredient, for a dose of 100 mg. The filling process also takes place inside a rigid-walled aseptic isolator using the same material sterilisation operation as in the previous examples.

The female syringes (1) pre-capped with the nozzle cap (8) to be used for filling are subjected to an ionisation process by a ring ioniser (2), thus ionising (a) the syringes (1) to remove the electrostatic charge inside them and in the sealing area. The cylinder (7) containing the ionised syringe (1) in this case is made of an insulating material. The syringe (1) is placed in the weighing cell (5), taring its weight to zero. Then the syringe (1) is filled with 100 mg ± 30% of PLGA by means of a nozzle (4) made of insulating material. The syringe (1) is continuously weighed during filling so that the system can be controlled to stop filling when the desired weight, in this case between 100 mg ± 30%, is reached.

After this process, the electrostatic charge inside the inner walls of the container (1) is measured and the measurement obtained is 785 volts.

After filling the female syringe (1) with the excipient, it is subjected to an ionisation process (c) using a needle ioniser (2), thus preventing the PLGA from adhering to the walls of the sealing area of the container (1).

After this process, the electrostatic charge inside the inner walls of the container (1) is measured and the measurement obtained is 580 volts.

After filling with the excipient and subsequent ionisation, the cylinder (7) with the syringe (1) filled with PLGA is placed in the next filling station (b) with the active ingredient Risperidone. The cylinder (7) with the syringe (1) is placed in the weighing cell (5), where it is tared before filling with the active ingredient, and then filling with Risperidone begins. The syringe (1) is continuously weighed during filling so that filling can be stopped once the desired weight has been reached.

After filling with the active ingredient, the cylinder (7) with the syringe (1) is again subjected to an ionisation process, where with the help of a needle ioniser (2), both the active ingredient and the excipient are prevented from adhering to the walls of the sealing area of the syringe (1). After this process, the electrostatic charge inside the inner walls of the container (1) is measured and the measurement obtained is 440 volts.

After this ionisation process, the syringe (1) passes to the sealing station (d) to be fitted with the stopper (6) and undergoes a further ionisation process. This process requires a rod ioniser (2) to ionise the PLGA and Risperidone that are adhered to the walls of the sealing area. After this process, the electrostatic charge inside the inner walls of the container (1) is measured and the measurement obtained is 197 volts.

The ionisation process ensures an optimum sealing and the total cleanliness of the sealing area, as well as preventing both PLGA and Risperidone from adhering to the stopper (6) used for sealing and to the walls of the container due to the electrostatic charges created by the friction created when the container is placed in the sealer.

Example 6: Filling of a 75 mg dose of Risperidone in cartridges or carpules.

In this other example, PLGA is used as excipient and Risperidone as active ingredient, for a dose of 75 mg. The filling process also takes place inside a rigid-walled aseptic isolator using the same material sterilisation operation as in the previous examples.

Both the PLGA used as excipient and the Risperidone used as active ingredient, as well as the cartridges, are delivered to the operators at the filling station (b) and loaded into their respective hoppers (3). The cartridges or carpules (1) to be used for filling are arranged under a stream of nitrogen or sterile carrier gas carrying ionised air molecules, a needle ioniser (2) is added to this process, and the cartridges or carpules (1) are ionised to eliminate the electrostatic charge inside them.

After this process, the electrostatic charge inside the inner walls of the container (1) is measured and the measurement obtained is 1285 volts.

The ionised cartridge or carpule (1) is directed to the filling station (b) with 75 mg ± 30% Risperidone. The cartridge (1) is placed in the weighing cell (5) face up, taring its weight to zero. Then the cartridge (1) is filled with Risperidone through the nozzle of the cartridge using a nozzle (4) or dispensing needle made of insulating material. The cartridge (1) is continuously weighed during filling so that the system can be controlled to stop filling when the desired weight is reached.

Once the cartridge (1) has been filled with the active ingredient, it is placed in the second filling station (b), this time with 100 mg ± 30% of the excipient PLGA. The cartridge (1) is placed in the weighing cell (5) where it is tared before being filled with this active ingredient, after which the filling with PLGA also starts from the nozzle. The cartridge (1) is continuously weighed during filling, so that filling can be stopped once the desired weight has been reached.

After filling with the PLGA excipient, the cartridge (1) undergoes a further ionisation process using a rod ioniser (2) and a stream of nitrogen or sterile carrier gas carrying ionised air molecules to displace the ions and act as a carrier for the insertion of the cartridge nozzle cover. These two processes prevent both the excipient and the active ingredient from adhering to the walls, thus achieving the sealing phenomenon.

After this process, the electrostatic charge inside the inner walls of the container (1) is measured and the measurement obtained is 1085 volts.

Example 7. Filling of a 400 mg dose of Risperidone in a pre-capped female syringe.

In this example, PLGA is used as excipient and Risperidone as active ingredient, for a dose of 400 mg. The filling process also takes place inside a rigid-walled aseptic isolator using the same material sterilisation operation as in the previous examples.

The female syringes (1) to be used for filling are subjected to an ionisation process by means of a ring ioniser (2), so that the syringes (1) are ionised (a) to eliminate the electrostatic charge inside them and in the sealing area. The cylinder (7) containing the ionised syringe (1) in this case is made of an insulating material. The syringe (1) is placed in the weighing cell (5) upside down since it is previously closed with a stopper (6), taring its weight to zero. After this, the syringe (1) is filled through the nozzle with 100 mg + 30% PLGA, using a nozzle (4) made of insulating material. The syringe (1) is continuously weighed during filling so that the system can be controlled to stop filling when the desired weight, in this case between 100 mg ± 30%, is reached.

After this process, the electrostatic charge inside the inner walls of the container (1) is measured and the measurement obtained is 735 volts.

After filling the female syringe (1) with the excipient, it is subjected to an ionisation process (c) using a rod ioniser (2), thus preventing the PLGA from adhering to the walls of the container (1) near the nozzle.

After this process, the electrostatic charge inside the inner walls of the container (1) is measured and the measurement obtained is 530 volts.

After filling with the excipient and subsequent ionisation, the cylinder (7) with the syringe (1) filled with PLGA is placed in the next filling station (b) with the active ingredient Risperidone. The cylinder (7) with the syringe (1) is placed in the weighing cell (5), where it is tared before filling with the active ingredient, and then filling with Risperidone begins. The syringe (1) is continuously weighed during filling so that filling can be stopped once the desired weight has been reached.

After filling with the active ingredient, the cylinder with the syringe (1) is again subjected to an ionisation process, where, with the help of a rod ioniser (2), both the active ingredient and the excipient are prevented from adhering to the walls of the syringe (1). After this process, the electrostatic charge inside the inner walls of the container (1) is measured and the measurement obtained is 215 volts.

The ionisation process provides an optimum sealing and the precise dosage required.

**Claims**

1. A procedure for filling pharmaceutical containers (1) with at least one solid and sealing them under sterile conditions, comprising the steps of:

   a) providing a pharmaceutical container (1) having walls and a bottom,
   b) dispensing the solid into the pharmaceutical container (1) by means of a dispensing needle (4), gravimetrically checking the weight of solid dispensed into the container (1); and
   c) sealing the pharmaceutical container with a stopper (6),

   wherein, **characterised in that**,

   in at least one of steps a), b) and c), or in a plurality thereof in any combination, the static electric charges on the inside walls of the container (1), on the solid dispensed inside the container, and/or on any parts in contact with the inside walls of the container or with the solid dispensed inside the container, are neutralised by means of an ioniser (2) to which an ionisation potential is applied such that the electrostatic charge inside the container (1) after each ionisation is less than 2000 volts,
and wherein, between step b) and step c), an additional static electric charge ionisation is carried out inside the pharmaceutical container (1).

2. A procedure according to claim 1, wherein in step a) the ionisation is carried out with the pharmaceutical container (1) empty.

3. A procedure according to claims 1 or 2, wherein the ioniser(s) (2) are located on the outside of the pharmaceutical container (1).

4. A procedure according to any one of claims 1 to 3, wherein the ioniser(s) (2) are located inside of the pharmaceutical container (1).

5. A procedure according to any one of claims 1 to 4, wherein, in step c), the ionisation is carried out before and/or during sealing

6. A procedure according to any one of the preceding claims, **characterised in that** the static electrical charges are less than 1000 volts.

7. A procedure according to any one of the preceding claims, **characterised in that** the static electrical charges are less than 500 volts.

8. A procedure according to any one of the preceding claims, **characterised in that** the static electrical charges are less than 200 volts.

9. A procedure according to any one of the preceding claims wherein, in step b), the tip of the dispensing end of the dispensing needle (4) is at a height h of 1 to 3 mm above the surface of the solid deposited on the bottom of the container (1).

10. A procedure according to any one of the preceding claims, wherein, in step b), the container (1) is in a fixed position throughout the filling stage while the dispensing needle (4) is a movable element that moves upwards as the filling stage progresses in order to maintain the distance h between the dispensing end of the dispensing needle and the surface of the solid deposited on the bottom of the container (1).

11. A procedure according to any one of the preceding claims, wherein step c) is carried out under vacuum

12. A procedure according to any one of the preceding claims, wherein in at least one of steps a), b) and c) or in a plurality thereof in any combination, a sterile carrier gas stream such as $N_2$ or sterile compressed air is applied inside the pharmaceutical container (1).

13. A procedure according to any one of the preceding claims, wherein the ioniser (2) is selected from the group consisting of ring, rod, gun, curtain, blade, gun, needle or filter ionisers, and isolators with an ioniser on the top thereof.

14. A procedure according to any one of the preceding claims, wherein the dispensing needle (d) dispenses the solid contained in a hopper (c), and both the hopper (c) and the dispensing needle (d) are made of a non-conductive material.

15. A procedure according to any one of the preceding claims, wherein the pharmaceutical container (1) is selected from the group consisting of a male syringe, a female syringe, a needle syringe, a vial, a capsule, an ampoule, a single-dose device, a cartridge, an inhaler, a bottle, a blister pack, a sachet, a bag, a test tube, and an Eppendorf® tube.

16. A procedure according to any one of the preceding claims, wherein the dispensing needle (4) is provided with a containment element to prevent the powder from being dispersed above the level of the dispensing tip or end of the dispensing needle during filling.

17. A procedure according to any one of the preceding claims, **characterised in that** it is carried out in an aseptic environment in an area with unidirectional air flow.

18. A procedure according to any one of the preceding claims, **characterised in that** it is carried out in an isolator.

19. A procedure according to any one of the preceding claims **characterised in that** prior to step b) a sterilisation of the isolator is carried out with nebulised or vaporised hydrogen peroxide or a mixture of hydrogen peroxide and peracetic acid.

20. A procedure according to any one of the preceding claims that is implemented in computer executable software.

**Patentansprüche**

1. Verfahren zum Abfüllen pharmazeutischer Behälter (1) mit mindestens einem Feststoff und dessen Verschluss unter sterilen Bedingungen, umfassend die folgenden Schritte:

   a) Bereitstellen eines pharmazeutischen Behälters (1) mit Wänden und einem Boden,
   b) Abgeben des Feststoffs in den pharmazeutischen Behälter (1) mittels einer Dosiernadel (4), gravimetrisches Überprüfen des Gewichts des in den Behälter (1) abgegebenen Feststoffs; und
   c) Verschließen des pharmazeutischen Behälters mit einem Stopfen (6),
   wobei, **dadurch gekennzeichnet**, in mindestens einem der Schritte a), b) und c) oder in einer Vielzahl davon in einer beliebigen Kombination die statischen elektrischen Ladungen an den Innenwänden des Behälters (1), an dem im Inneren des Behälters abgegebenen Feststoff und/oder an allen Teilen, die mit den Innenwänden des Behälters oder mit dem im Inneren des Behälters abgegebenen Feststoff in Kontakt stehen, mittels eines Ionisators (2) neutralisiert werden, an den ein Ionisierungspotential angelegt ist, so dass die elektrostatische Ladung im Inneren des Behälters (1) nach jeder Ionisierung weniger als 2000 Volt beträgt, und wobei zwischen Schritt b) und Schritt c) eine zusätzliche statische elektrische Ladungsionisierung innerhalb des pharmazeutischen Behälters (1) durchgeführt wird.

2. Verfahren nach Anspruch 1, wobei in Schritt a) die Ionisierung bei leerem pharmazeutischem Behälter (1) durchgeführt wird.

3. Verfahren nach Anspruch 1 oder 2, wobei sich der/die Ionisator(en) (2) an der Außenseite des pharmazeutischen Behälters (1) befindet/befinden.

4. Verfahren nach einem der Ansprüche 1 bis 3, wobei sich der/die Ionisator(en) (2) im Inneren des pharmazeutischen Behälters (1) befindet/befinden.

5. Verfahren nach einem der Ansprüche 1 bis 4, wobei in Schritt c) die Ionisierung vor und/oder während dem Verschluss durchgeführt wird.

6. Verfahren nach einem der vorstehenden Ansprüche, **dadurch gekennzeichnet, dass** die statischen elektrischen Ladungen weniger als 1000 Volt betragen.

7. Verfahren nach einem der vorstehenden Ansprüche, **dadurch gekennzeichnet, dass** die statischen elektrischen Ladungen weniger als 500 Volt betragen.

8. Verfahren nach einem der vorstehenden Ansprüche, **dadurch gekennzeichnet, dass** die statischen elektrischen Ladungen weniger als 200 Volt betragen.

9. Verfahren nach einem der vorstehenden Ansprüche, wobei sich in Schritt b) die Spitze des Dosierungsendes der Dosiernadel (4) in einer Höhe h von 1 bis 3 mm über der Oberfläche des auf dem Boden des Behälters (1) abgelagerten Feststoffs befindet.

10. Verfahren nach einem der vorstehenden Ansprüche, wobei sich der Behälter (1) in Schritt b) während der gesamten Füllphase in einer festen Position befindet, während die Dosiernadel (4) ein bewegliches Element ist, das sich im Verlauf der Füllphase nach oben bewegt, um den Abstand h zwischen dem Dosierende der Dosiernadel und der Oberfläche des auf dem Boden des Behälters (1) abgelagerten Feststoffs beizubehalten.

11. Verfahren nach einem der vorstehenden Ansprüche, wobei Schritt c) unter Vakuum durchgeführt wird.

12. Verfahren nach einem der vorstehenden Ansprüche, wobei in mindestens einem der Schritte a), b) und c) oder in einer Vielzahl davon in einer beliebigen Kombination ein steriler Trägergasstrom wie N2 oder sterile Druckluft in den pharmazeutischen Behälter (1) eingeleitet wird.

13. Verfahren nach einem der vorstehenden Ansprüche, wobei der Ionisator (2) aus der Gruppe ausgewählt ist, bestehend aus Ring-, Stangen-, Pistolen-, Vorhang-, Klingen-, Nadel- oder Filterionisatoren und Isolatoren mit einem Ionisator auf der Oberseite davon.

14. Verfahren nach einem der vorstehenden Ansprüche, wobei die Dosiernadel (d) den in einem Trichter (c) enthaltenen Feststoff abgibt und sowohl der Trichter (c) als auch die Dosiernadel (d) aus einem nicht leitfähigen Material hergestellt sind.

15. Verfahren nach einem der vorstehenden Ansprüche, wobei der pharmazeutische Behälter (1) aus der Gruppe ausgewählt ist, bestehend aus einer Spritze mit Außengewinde, einer Spritze mit Innengewinde, einer Nadelspritze, einem Fläschchen, einer Kapsel, einer Ampulle, einer Einzeldosisvorrichtung, einer Patrone, einem Inhalator, einer Flasche, einer Blisterpackung, einem Portionstütchen, einem Beutel, einem Reagenzglas und einem Eppendorf®-Röhrchen.

16. Verfahren nach einem der vorstehenden Ansprüche, wobei die Dosiernadel (4) mit einer Sicherheitshülle versehen ist, um zu verhindern, dass das Pulver beim Befüllen über das Niveau der Dosierspitze oder des Endes der Dosiernadel hinaus, verstreut wird.

17. Verfahren nach einem der vorstehenden Ansprüche, **dadurch gekennzeichnet, dass** es in einer aseptischen Umgebung in einem Bereich mit unidirektionaler Luftströmung durchgeführt wird.

18. Verfahren nach einem der vorstehenden Ansprüche, **dadurch gekennzeichnet, dass** es in einem Isolator durchgeführt wird.

19. Verfahren nach einem der vorstehenden Ansprüche, **dadurch gekennzeichnet, dass** vor Schritt b) eine Sterilisation des Isolators mit vernebeltem oder verdampftem Wasserstoffperoxid oder einem Gemisch aus Wasserstoffperoxid und Peressigsäure durchgeführt wird.

20. Verfahren nach einem der vorstehenden Ansprüche, das in computerausführbarer Software implementiert ist.

**Revendications**

1. Procédé pour le remplissage d'au moins un solide dans des contenants pharmaceutiques (1) et le scellement de ceux-ci dans des conditions stériles, comprenant les étapes :

   a) fournir un contenant pharmaceutique (1) ayant des parois et un fond,
   b) distribuer le solide dans le contenant pharmaceutique (1) au moyen d'une aiguille de distribution (4), en vérifiant par gravité le poids du solide distribué dans le contenant (1) ; et
   c) sceller le contenant pharmaceutique avec un bouchon (6),
   dans lequel, dans au moins l'une des étapes a), b) et c), ou dans une pluralité de celles-ci dans une combinaison quelconque, les charges électriques statiques sur les parois internes du contenant (1), sur le solide distribué à l'intérieur du contenant, et/ou sur toute partie en contact avec les parois internes du contenant ou avec le solide distribué à l'intérieur du contenant, sont neutralisées au moyen d'un ioniseur (2) auquel est appliqué un potentiel d'ionisation tel que la charge électrostatique à l'intérieur du contenant (1) après chaque ionisation est inférieure à 2000 volts,
   et dans lequel, entre l'étape b) et l'étape c), une ionisation de charge électrique statique supplémentaire est effectuée à l'intérieur du contenant pharmaceutique (1).

2. Procédé selon la revendication 1, dans lequel, à l'étape a), l'ionisation est effectuée avec le contenant pharmaceutique (1) vide.

3. Procédé selon la revendication 1 ou 2, dans lequel le ou les ioniseurs (2) sont situés à l'extérieur du contenant pharmaceutique (1).

4. Procédé selon l'une quelconque des revendications 1 à 3, dans lequel le ou les ioniseurs (2) sont situés à l'intérieur du contenant pharmaceutique (1).

5. Procédé selon l'une quelconque des revendications 1 à 4, dans lequel, à l'étape c), l'ionisation est effectuée avant et/ou pendant le scellement.

6. Procédé selon l'une quelconque des revendications précédentes, **caractérisé en ce que** les charges électriques statiques sont inférieures à 1000 volts.

7. Procédé selon l'une quelconque des revendications précédentes, **caractérisé en ce que** les charges électriques statiques sont inférieures à 500 volts.

8. Procédé selon l'une quelconque des revendications précédentes, **caractérisé en ce que** les charges électriques statiques sont inférieures à 200 volts.

9. Procédé selon l'une quelconque des revendications précédentes, dans lequel, à l'étape b), la pointe de l'extrémité de distribution de l'aiguille de distribution (4) se trouve à une hauteur h de 1 à 3 mm au-dessus de la surface du solide déposé sur le fond du contenant (1).

10. Procédé selon l'une quelconque des revendications précédentes, dans lequel, à l'étape b), le contenant (1) est dans une position fixe tout au long de l'étape de remplissage tandis que l'aiguille de distribution (4) est un élément mobile qui se déplace vers le haut au fur et à mesure que l'étape de remplissage progresse afin de maintenir la distance h entre l'extrémité de distribution de l'aiguille de distribution et la surface du solide déposé sur le fond du contenant (1).

11. Procédé selon l'une quelconque des revendications précédentes, dans lequel l'étape c) est effectuée sous vide.

12. Procédé selon l'une quelconque des revendications précédentes, dans lequel, dans au moins l'une des étapes a), b) et c) ou dans une pluralité de celles-ci dans une combinaison quelconque, un flux de gaz porteur stérile tel que N2 ou de l'air comprimé stérile est appliqué à l'intérieur du contenant pharmaceutique (1).

**13.** Procédé selon l'une quelconque des revendications précédentes, dans lequel l'ioniseur (2) est choisi dans le groupe constitué d'ioniseurs à anneau, à tige, à pistolet, à rideau, à lame, à pistolet, à aiguille ou à filtre, et d'isolateurs ayant un ioniseur sur le dessus.

**14.** Procédé selon l'une quelconque des revendications précédentes, dans lequel l'aiguille de distribution (d) distribue le solide contenu dans une trémie (c), et la trémie (c) et l'aiguille de distribution (d) sont toutes deux faites d'un matériau non conducteur.

**15.** Procédé selon l'une quelconque des revendications précédentes, dans lequel le contenant pharmaceutique (1) est choisi dans le groupe constitué d'une seringue mâle, d'une seringue femelle, d'une seringue à aiguille, d'une fiole, d'une capsule, d'une ampoule, d'un dispositif à dose unique, d'une cartouche, d'un inhalateur, d'un flacon, d'un blister, d'un sachet, d'un sac, d'un tube à essai et d'un tube Eppendorf®.

**16.** Procédé selon l'une quelconque des revendications précédentes, dans lequel l'aiguille de distribution (4) est pourvue d'un élément de confinement pour empêcher la poudre d'être dispersée au-dessus du niveau de la pointe de distribution ou de l'extrémité de l'aiguille de distribution pendant le remplissage.

**17.** Procédé selon l'une quelconque des revendications précédentes, **caractérisé en ce qu'**il est effectué dans un environnement aseptique dans une zone à flux d'air unidirectionnel.

**18.** Procédé selon l'une quelconque des revendications précédentes, **caractérisé en ce qu'**il est effectué dans un isolateur.

**19.** Procédé selon l'une quelconque des revendications précédentes, **caractérisé en ce qu'**avant l'étape b), une stérilisation de l'isolateur est effectuée avec du peroxyde d'hydrogène nébulisé ou vaporisé ou un mélange de peroxyde d'hydrogène et d'acide peracétique.

**20.** Procédé selon l'une quelconque des revendications précédentes qui est mis en œuvre dans un logiciel exécutable par ordinateur.

FIG.1

FIG.2

FIG.3

FIG.4

EP 3 875 377 B1

FIG.5

FIG.6

$n = 1-\infty$

(3)

(4)

(2)

(6)

(7)

(1)

(6)

(5)

(2)

(1)

(6)

# FIG.7

EP 3 875 377 B1

**REFERENCES CITED IN THE DESCRIPTION**

**Patent documents cited in the description**

- US 20160200461 A1
- EP 2711096 A2
- EP 2711096 A
- JP 2005001818 A
- CN 203265193 U
- WO 2016185230 A2
- WO 2018017561 A